# FASCICULE DE BREVET EUROPEEN

(11) **EP 1 403 375 B1**
(45) Date de publication et mention de la délivrance du brevet: **10.10.2007**
(21) Numéro de dépôt: 03025194.6
(22) Date de dépôt: 28.12.1995
(51) Int. Cl.: C12N 15/86, C07K 14/055, C07K 14/125, C07K 14/165, A61K 39/255, A61K 39/17, A61K 39/215

(54) **Vaccin vivant recombinant aviaire, utilisant comme vecteur un virus herpès aviaire**
Rekombinanter lebender Vogel-Impfstoff, der ein Hühne-Herpesvirus Vektor gebraucht
Recombinant live vaccine, using avian herpes virus as vector

(30) Priorité: 30.12.1994 FR 9416017
(43) Date de publication de la demande: 31.03.2004
(62) Demande divisionnaire de: 95402970.8
(73) Titulaire: MERIAL, 69007 Lyon (FR)
(72) Inventeur: Audonnet, Jean-Christophe, 69006 Lyon (FR); Bublot, Michel, 69630 Chaponost (FR); Darteil, Raphael, 59800 Lille (FR); Duinat, Carole, 69007 Lyon (FR); Laplace, Eliane, 69600 Oullins (FR); Riviere, Michel, 69130 Ecully (FR)
(74) Mandataire: Nargolwalla, Cyra

(56) Documents cités:
- WO-A-93/25665
- FR-A- 2 697 534
- DARTEIL, R. ET AL.: "Herpesvirus of Turkey Recombinant Viruses expressing Infectious Bursal Disease Virus (IBDV) VP2 immunogen induce protection against an IBDV virulent challenge in chickens" VIROLOGY, vol. 211, no. 2, février 1995 (1995-02), pages 481-490, XP000601543 ORLANDO US
- BUCKMASTER, A.E. ET AL.: "Gene sequence and mapping data from Marek's Disease Virus and Herpesvirus of Turkeys: Implications for Herpesvirus classification" JOURNAL OF GENERAL VIROLOGY, vol. 69, no. 8, août 1988 (1988-08), pages 2033-2042, XP000117969

## Description

La présente invention a trait à l'utilisation pour la production de vaccins à usage aviaire de virus HVT (Herpes Virus of Turkey), dans lesquels a été insérée, par recombinaison génétique, au moins une séquence nucléotidique codant pour, et exprimant, un polypeptide antigénique d'un agent pathogène aviaire, dans des conditions visant à assurer une immunisation conduisant à une protection efficace de l'animal vacciné contre ledit agent pathogène.

On a déjà proposé un certain nombre de vecteurs viraux aviaires recombinants dans l'intention de vacciner les oiseaux contre des agents pathogènes aviaires, notamment des virus pathogènes, parmi lesquels figurent les virus de la maladie de Marek (MDV), de la maladie de Newcastle (NDV), de la laryngotrachéite infectieuse (ILTV), de la maladie de Gumboro (Infectious Bursal Disease, IBDV), de la bronchite infectieuse (IBV) et de l'anémie aviaire (CAV).

Les vecteurs viraux utilisés comprennent des avipox, en particulier le Fowlpox, (EP-A-0 517 292 ; H.-G. Heine et al., Arch. Virol. 1993. 131. 277-292 ; D.B. Boyle et al., Veterinary Microbiology 1994. 41. 173-181 ; C.D. Bayliss et al., Arch. Virol. 1991. 120. 193-205), les virus de Marek, notamment les sérotypes 2 et 3 (HVT) (WO-A-87 04463 ; WO-A-89 01040 ; WO-A-93 25665 ; EP-A-0 513 921 ; J. McMillen, Poultry Condemnation Meeting, October 1994, 359-363 ; P.J.A. Sondermeijer et al., Vaccine 1993. 11. 349-357 ; R.W. Morgan et al., Avian Diseases 1992. 36. 858-870, et 1993. 37. 1032-1040) ou encore les virus ILTV et l'adénovirus aviaire.

Lorsqu'ils sont utilisés pour la vaccination, ces virus recombinants induisent des protections de niveaux variables, généralement faibles ou partielles, même si, dans de rares cas particuliers, une protection importante peut être démontrée.

Parmi les protections les plus difficiles à assurer par des vaccins aviaires vivants recombinants, se trouve celle contre le virus de la maladie de Gumboro, ou virus IBDV. En effet, bien qu'il existe des vaccins vivants atténués ou inactivés classiques efficaces contre cette maladie, aucun vaccin vivant recombinant n'a encore fait preuve d'une efficacité convenable.

Le génome du virus de la maladie de Gumboro est constitué d'un ARN double brin. Le segment le plus grand (segment A) code pour une polyprotéine de 115 kDa secondairement clivée en trois protéines VP2 (41 kDa), VP4 (28 kDa), VP3 (32 kDa). VP4 serait une protéase intervenant dans la maturation de la polyprotéine 115 kDa. La position du site de clivage entre VP2 et VP4 n'est déterminée qu'approximativement (M. Jagadish, J. Virol. 1988. 62. 1084-1087). La protéine VP2 est un immunogène induisant des anticorps neutralisants et une protection contre la maladie de Gumboro.

On a déjà proposé d'insérer des gènes codant pour des protéines immunogènes d'IBDV dans divers vecteurs vivants: EP-A-0 517 292 (insertion de séquences codant pour VP2 ou la polyprotéine dans un avipox) ; C.D. Bayliss 1991, H.-G. Heine 1993 et D.B. Boyle 1994 *supra* (VP2 dans le Fowlpox).

Les virus de la maladie de Marek ont également été proposés dans WO-A-90 02802 et WO-A-90 02803 (sites d'insertion divers tels que gC, TK, RR1, RR2), dans les demandes de brevet français n° 90 03105 (RR2) et 90 11146 (US3), ainsi que, notamment, dans les demandes de brevet WO-A-87 04463 et WO-A-89 01040 (BamHl #16 et #19) et WO-A-93 25655 (US2).

R.J. Isfort et al. (Virology 1994. 203. 125-133) ont déterminé un certain nombre de sites d'intégration de rétrovirus dans le génome de HVT, sites qui sont situés dans les fragments de restriction BamH I F, A et I.

D'autres exemples de sites d'insertion dans le génome de HVT ont été proposés dans EP-A-0 431 668 (Région US) et EP 4 200 384 (gA).

FR-A-2 697 534 décrit un virus recombinant HVT exprimant une séquence codant pour la protéine VP2 d'IBDV insérée dans US2 (Short Unique Sequence, BaM HI A) sous le contrôle du promoteur IE d'HCMV. Ce virus recombinant exprime in vitro la protéine mais n'a pas été essayé in vivo dans un protocole vaccinal. DARTEIL, R. ET AL., VIROLOGY, vol. 211, no. 2, février 1995, pages 481-490, divulgue l'utilisation de deux virus HVT recombinants exprimant la protéine VP2 d'IBDV insérée dans locus non essentiel du génome HVT dans le fragment BamHi K1 soit dans la petite sous-unité du gène de la réductase de ribonucléotide (RR2) sans promoteur exogène (virus vHVT001), soit au locus du gène sous le contrôle du promoteur CMV Immediate Early (IE) humain, pour la production d'un vaccin vivant recombinant aviaire destiné à la vaccination des poussins de un jour ou des adultes contre le virus de la maladie de Gumboro (IBDV). Des poussins SPF d'un jour ont été vaccinés par injection intramusculaire unique avec des doses variables et éprouvés à l'âge de 21 jours par voie intra-oculaire avec un virus IBDV de la souche 52/70. Les résultats sont comparés avec ceux des animaux témoins non vaccinés ou vaccinés avec le vaccin inactivé GUMBORIFFA (ND) ont montré une protection par le virus recombinant vHVT002 capable d'induire la production d'anticorps anti-VP2 neutralisants et de réduire la mortalité ainsi que les lésions cliniques alors que vHVT001 n'offre qu'une protection partielle. La comparaison des deux virus recombinants HVT montre que le site d'insertion du gène exogène ainsi que le promoteur utilisé pour contrôler l'expression de ce gène sont deux paramètres importants.

Divers promoteurs, y compris ceux généralement disponibles dans le commerce, ont été utilisés dans les différentes constructions de l'art antérieur, parmi lesquels les promoteurs PRV gX, HCMV IE (CMV immediate early humain), herpès simplex alpha-4, FPV P.E/L (promoteur Fowlpox) (H. Heine et al., Arch. Virol. 1993. 131. 277-292), P7.5 (C. Bayliss et al., Arch. Virol. 1991. 120. 193-205) et P11 du virus de la vaccine (D. Boyle et al. Vet. Microb. 1994. 41. 173-181), provenant de la séquence LTR du virus RSV (Rous Sarcoma Virus), SV40 précoce, ainsi que des promoteurs MDV ou HVT, tels que les promoteurs des gènes gB, gC, TK, RR2, etc., sans qu'ait pu apparaître une règle, notamment dans le cas des constructions dans HVT. Les séquences de certains promoteurs peuvent inhiber la réplication des vecteurs recombinants HVT ou MDV (D.R. Marshall et al., J. Vir. Meth. 1992. 40. 195-204 et Virology 1993. 195. 638-648). Parmi les promoteurs cités, un certain nombre, comme par exemple SV40, LTR RSV et PRV gX, ont montré une certaine efficacité, de même que certains promoteurs propres de certains gènes des virus Marek, notamment de sérotype 3. Il reste un besoin dans l'art de développer d'autres vecteurs HVT pour la vaccination *in ovo,* pour les poussins de un jour et chez l'adulte, afin d'offrir un éventail vaccinal plus large et développer une protection appropriée contre divers pathogènes aviaires.

L'invention a permis de mettre au point un vaccin vivant recombinant à base d'un vecteur HVT dans lequel est insérée au moins une séquence codant pour un immunogène aviaire, en particulier la protéine VP2 d'IBDV. Un tel vaccin incorporant une séquence codant pour VP2 doit assurer une protection satisfaisante des animaux contre la maladie de Gumboro, à savoir protection vis-à-vis de la mortalité et des lésions de la bourse de Fabricius.

La présente invention a pour objet l'utilisation pour la production d'un vaccin vivant recombinant aviaire d'un virus herpès aviaire HVT comprenant au moins une séquence nucléotidique codant pour, et exprimant, un polypeptide antigénique d'un agent pathogène aviaire, insérée dans le fragment de restriction BamHl I de HVT à savoir dans l'une des trois régions intergéniques 1, 2 et 3, notamment sous le contrôle du promoteur CMV immediate early. Le fragment BamHI de HVT est présenté par A.E. Buckmaster dans J. Gen. Virol. 1988. 69. 2033-2042.

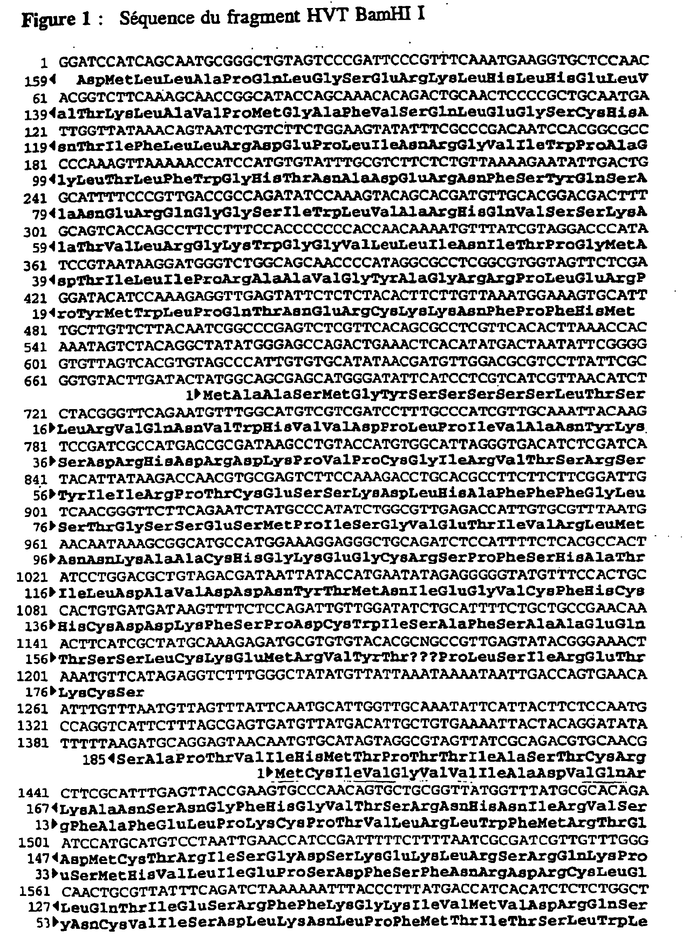

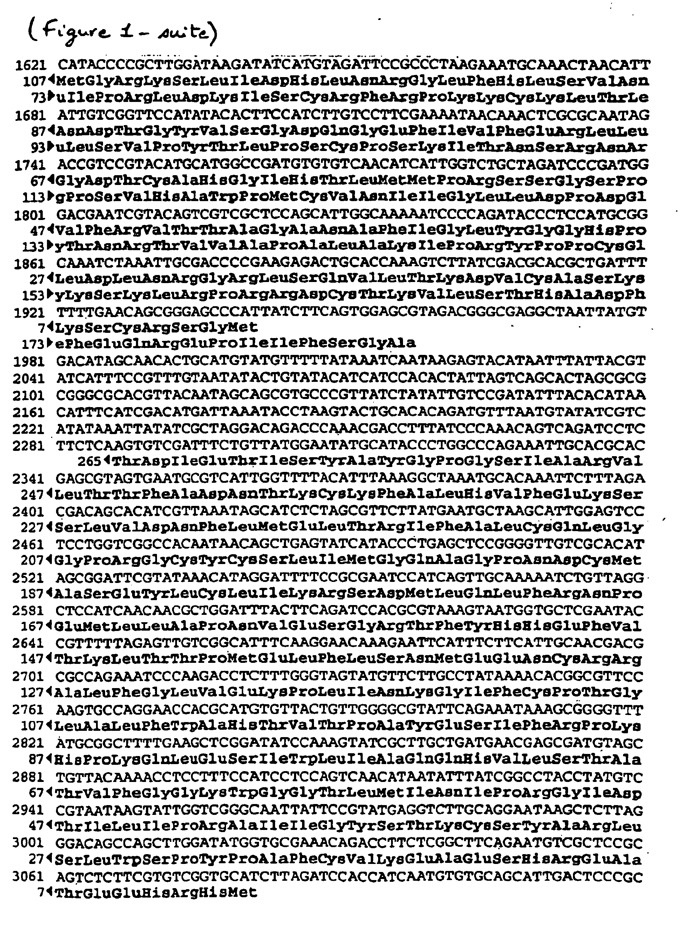

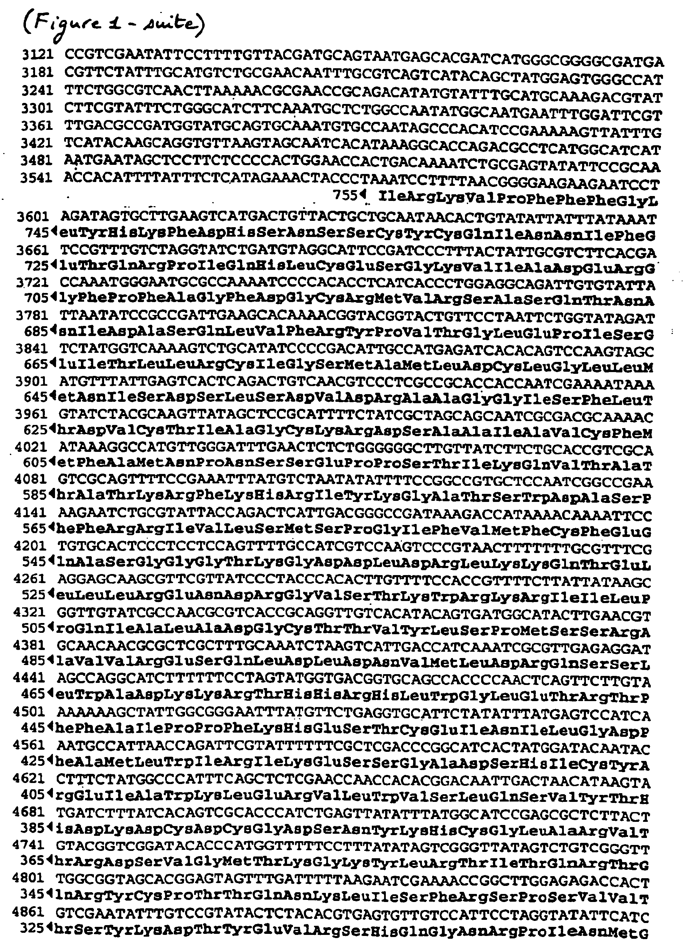

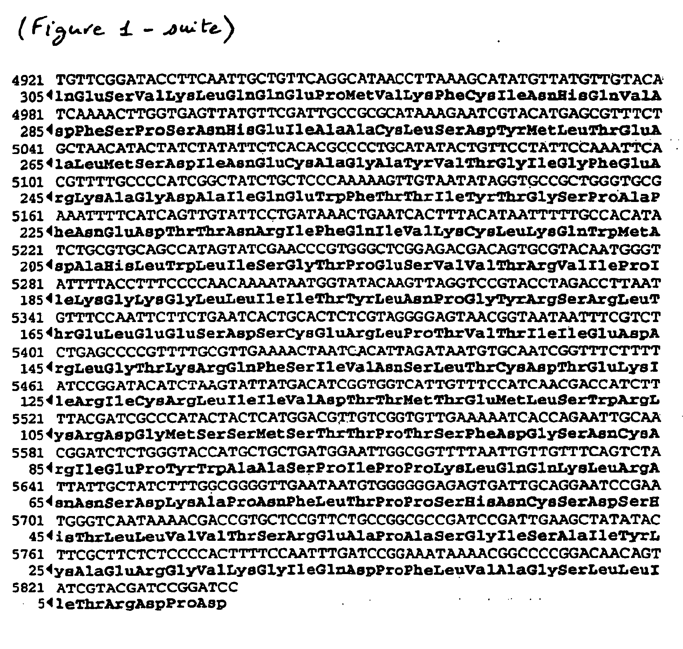

Ces zones intergéniques correspondent, par référence à la souche HVT particulière à laquelle la séquence nucléotidique de la figure 1 correspond, aux nucléotides de la figure 1 1213 à 1383 pour l'intergène 1, 1942 à 2283 pour l'intergène 2 et 3082 à 3569 pour l'intergène 3.

Par insertion dans la région d'insertion, on entend notamment insertion sans délétion ou avec délétion de quelques bases pour les zones intergéniques.

Par promoteur CMV immediate early (IE), on entend le fragment donné dans les exemples ainsi que ses sous-fragments conservant la même activité promotrice.

Le promoteur CMV IE peut être le promoteur humain (HCMV IE) ou le promoteur murin (MCMV IE), ou encore un promoteur CMV IE d'une autre origine, par exemple du rat ou du cochon d'Inde.

La séquence nucléotidique insérée dans le vecteur Marek pour être exprimée peut être toute séquence codant pour un polypeptide antigénique, d'un agent pathogène aviaire, capable, une fois exprimé dans les conditions favorables procurées par l'invention, d'assurer une immunisation conduisant à une protection efficace de l'animal vacciné contre l'agent pathogène. On pourra donc insérer, dans les conditions de l'invention, les séquences nucléotidiques codant pour les antigènes d'intérêt pour une maladie donnée.

Les vaccins obtenus selon l'invention pourront être destinés à la vaccination *in ovo,* des poussins d' 1 jour ou plus et des adultes.

L'invention peut notamment être utilisée pour l'insertion d'une séquence nucléotidique codant convenablement pour le polypeptide VP2 du virus IBDV. On obtient ainsi un vaccin vivant recombinant assurant, en plus d'une protection contre la maladie de Marek, une protection satisfaisante contre la maladie de Gumboro. Si on le souhaite, on peut aussi insérer une séquence codant pour un autre antigène d'IBDV, tel que VP3 ou encore la polyprotéine VP2 + VP4 + VP3, ces autres possibilités n'étant pas préférées.

Le vaccin recombinant contre la maladie de Gumboro se présentera de préférence sous de 10 à 10⁴ pfu/dose.

D'autres cas préférés de l'invention sont l'insertion de séquences nucléotidiques codant pour des antigènes du virus de la maladie de Marek, en particulier gènes gB, gC, gD, et gH+gL (WO-A-90 02803), du virus de la maladie de Newcastle, en particulier gènes F et HN, du virus de la bronchite infectieuse (IBV), en particulier gènes S et M (M. Binns et al., J. Gen. Virol. 1985. 66. 719-726 ; M. Boursnell et al., Virus Research 1984. 1. 303-313), du virus de l'anémie aviaire (CAV), en particulier VP1 (52 kDa) + VP2 (24 kDa) (N.H.M. Noteborn et al., J. Virol. 1991. 65. 3131-3139) et du virus de la laryngotrachéite infectieuse (ILTV), en particulier gB (WO-A-90 02802), gC, gD et gH +gL.

Les doses seront de préférence les mêmes que celles pour le vaccin de Gumboro.

Selon un développement avantageux de l'invention, on associe au promoteur CMV IE un autre promoteur de sorte que les promoteurs aient des sens de transcription opposés, ce qui permet d'insérer, dans la zone d'insertion, deux séquences nucléotidiques, l'une sous la dépendance du promoteur CMV IE, l'autre sous celle du promoteur associé. Cette construction est remarquable par le fait que la présence du promoteur CMV IE, et notamment de sa partie activatrice (enhancer), active la transcription induite par le promoteur associé. Un promoteur associé préféré est le promoteur Marek RNA1.8, dont l'activité de transcription s'est révélée multipliée environ par 4,4 dans ces conditions.

Un cas intéressant de l'invention est un vaccin comprenant une séquence nucléotidique codant pour VP2 d'IBDV sous le contrôle de CMV IE et une séquence nucléotidique codant pour un antigène d'une autre maladie aviaire, notamment celles citées plus haut, sous le contrôle de l'autre promoteur.

On peut aussi monter tête-bêche deux promoteurs CMV IE d'origines différentes.

Le promoteur RNA1.8 pourra aussi être utilisé seul à la place du promoteur CMV IE, notamment pour des vaccins contre la maladie de Marek, la maladie de Newcastle, la laryngotrachéite infectieuse, la bronchite infectieuse et l'anémie aviaire.

L'invention va être maintenant décrite plus en détail à l'aide d'exemples de réalisation non limitatifs, pris en référence au dessin, dans lequel :
Liste des figures et des séquences pour les constructions dans les sites intergéniques
   - **Figure 1 :**: Séquence du fragment HVT BamHI I
   - **Figure 2** :: plasmide pEL039
   - **Figure 3** :: plasmide pEL077
   - **Figure 4** :: plasmide pEL079
   - **Figure 5** :: plasmide pEL076
   - **Figure 6** :: plasmide pEL078
   - **Figure 7 :**: plasmide pEL054
   - **Figure 8 :**: plasmide pEL055
   - **Figure 9** :: plasmide pEL062
   - **Figure 10 :**: plasmide pEL066
   - **Figure 11** :: plasmide pEL022
   - **Figure 12** :: plasmide pEL023
   - **Figure 13** :: plasmide pEL024
   - **Figure 14 :**: plasmide pCMVβ
   - **Figure 15** :: plasmide pEL026
   - **Figure 16** :: plasmide pEL090
   - **Figure 17** :: plasmide pCD002
   - **Figure 18** :: plasmide pCD009
   - **Figure 19** :: plasmide pEL068
   - **Figure 20** :: plasmide pEL070
   - **Figure 21** :: plasmide pEL091
   - **Figure 22** :: plasmide pCD011
   - **Figure 23** :: plasmide pCD020
   - **Figure 24** :: plasmide pEL092
   - **Figure 25** :: Séquence du gène NDV HN
   - **Figure 26** :: plasmide pEL028
   - **Figure 27** :: plasmide pEL029bis
   - **Figure 28** :: plasmide pEL030
   - **Figure 29** :: plasmide pEL032
   - **Figure 30** :: plasmide pEL093
   - **Figure 31** :: plasmide pEL033
   - **Figure 32** :: plasmide pEL034
   - **Figure 33** :: plasmide pEL094
   - **Figure 34** :: Séquence du promoteur MDV RNA 1.8 kpb
   - **Figure 35** :: plasmide pBS002
   - **Figure 36** :: plasmide pEL069
   - **Figure 37** :: plasmide pEL080
   - **Figure 38** :: plasmide pEL081
   - **Figure 39** :: plasmide pEL095
   - **Figure 40** :: plasmide pEL098
Liste des séquences SEQ ID pour les constructions dans les sites intergéniques
   - **SEQ ID N° 1**: Séquence du fragment HVT BamHI 1
   - **SEQ ID N° 2**: Oligonucléotide EL102
   - **SEQ ID N° 3**: Oligonucléotide EL161
   - **SEQ ID N° 4**: Oligonucléotide EL147
   - **SEQ ID N° 5**: Oligonucléotide EL162.
   - **SEQ ID N° 6**: Oligonucléotide EL154
   - **SEQ ID N° 7**: Oligonucléotide EL163
   - **SEQ ID N° 8**: Oligonucléotide EL164
   - **SEQ ID N° 9**: Oligonucléotide EL165
   - **SEQ ID N° 10**: Oligonucléotide EL132
   - **SEQ ID N° 11**: Oligonucléotide EL133
   - **SEQ ID N° 12**: Oligonucléotide MB070
   - **SEQ ID N° 13**: Oligonucléotide MB071
   - **SEQ ID N° 14**: Oligonucléotide CD001
   - **SEQ ID N° 15**: Oligonucléotide CD002
   - **SEQ ID N° 16**: Oligonucléotide CD003
   - **SEQ ID N° 17**: Oligonucléotide CD004
   - **SEQ ID N° 18**: Séquence du gène NDV HN
   - **SEQ ID N° 19**: Oligonucléotide EL071
   - **SEQ ID N° 20**: Oligonucléotide EL073
   - **SEQ ID N° 21**: Oligonucléotide EL074
   - **SEQ ID N° 22**: Oligonucléotide EL075
   - **SECS ID N° 23**: Oligonucléotide EL076
   - **SEQ ID N° 24**: Oligonucléotide EL077
   - **SEQ ID N° 25**: Séquence du promoteur MDV RNA 1.8 kpb
   - **SEQ ID N° 26**: Oligonucléotide MB047
   - **SEQ ID N° 27**: Oligonucléotide MB048
   - **SEQ ID N° 28**: Oligonucléotide MB072

### 2. EXEMPLES

Toutes les constructions de plasmides ont été réalisées en utilisant les techniques standards de biologie moléculaire décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention ont été isolés en utilisant le kit "Geneclean" (BI0101 Inc. La Jolla, CA).

Le virus utilisé comme virus parental est la souche FC126 de l'herpèsvirus de la dinde (HVT) isolé par le Dr. Witter du Regional Poultry Research Laboratory (USDA, East Lansing, Michigan), dans un troupeau de dindes de 23 semaines (Witter R.L. et al. Am. J. Vet. Res. 1970. 31. 525-538). Les conditions de culture de ce virus sont celles décrites par ailleurs (demande de brevet français 90 03105).

### Exemple 1: Extraction de l'ADN du virus de la maladie de Marek:

Le sang total d'un poulet éprouvé à 7 jours avec la souche MDV RB1B est récolté avec une seringue sur anticoagulant (solution d'héparine à 100 Ul/ml) 14 jours après infection. Ce sang est ensuite centrifugé à 30 g pendant 15 minutes à température ambiante. Le plasma ainsi que le "buffy-coat" sont prélevés et dilués dans du PBS stérile pour avoir un volume final de 10 ml. Après une centrifugation de 15 minutes à 150 g, le culot cellulaire est resuspendu dans 2 ml de milieu de culture 199 (Gibco-BRL Cat# 042-01183M) contenant 2 % de sérum de veau foetal (SVF).

L'ADN total des lymphocytes infectés est ensuite extrait selon la technique décrite par R. Morgan et al. (Avian Diseases. 1990. 34. 345-351) et peut directement servir de matrice pour les expériences de PCR. Pour le clonage de fragments génomiques du virus MDV, la souche RB1 B a été cultivée sur CEP et l'ADN viral a été préparé à partir de particules virales purifiées comme décrit par Lee Y. et al. (J. Gen. Virol. 1980. 51. 245-253).

### Exemple 2: Préparation de l'ADN génomique du virus MCMV (Mouse CyloMegaloVirus)

Le virus MCMV souche Smith a été obtenu de l'American Type Culture Collection, Rockville, Maryland, USA (ATCC N° VR-194). Ce virus a été cultivé sur cellules d'embryon de souris Balb/C et l'ADN viral de ce virus a été préparé comme décrit par Ebeling A. et al. (J. Virol. 1983. 47. 421 -433).

### Exemple 3: Préparation de l'ADN génomique du virus HVT pour les expériences de transfection:

L'ADN viral utilisé pour les expériences de transfection a été préparé selon la technique décrite par R. Morgan et al. (Avian Diseases. 1990. 34. 345-351) à partir d'une culture de CEP secondaires (CEP II) infectées avec la souche FC126 du virus HVT.

### Exemple 4: Description du fragment BamHl I

Le fragment BamHl 1 de 5,8 kpb du virus HVT souche FC126 (Igarashi T. et al. J. Gen. Virol. 1989. 70. 1789-1804) a été isolé par Geneclean et cloné dans le site BamHl du vecteur pBS-SK + pour donner le plasmide pEL037. La séquence de ce fragment a été établie en totalité (5838 pb) (figure 1 et SEQ ID N° 1). 6 cadres ouverts de lecture (= "open reading frames = ORFs") ont été identifiés sur cette séquence. L'étude des protéines potentiellement codées par ces ORFs a révélé que certaines de ces protéines présentaient une homologie avec des protéines codées par des ORFs présentes chez d'autres alphaherpèsvirus. La première ORF (ORF1) (position 676 à position 1209 sur SEQ ID N° 1) présente une homologie avec les ORFs HSV-1 UL55, EHV-1 gène 4 et VZV gène 5, et code pour une protéine théorique HVT UL55 de 178 acides aminés (aa). L'ORF2 est située de la position 1941 à la position 1387 sur la séquence SEQ ID N° 1 et code pour une protéine de 185 aa homologue de la protéine codée par l'ORF EHV-1 gène 3. L'ORF3 est incomplète. Elle est située de la position 5838 à 3573 sur SEQ ID N° 1 et elle présente une homologie avec l'ORF 21 de MDV (Ross N. et al. Virus Genes. 1993. 7. 33-51). Trois autres ORFs identifiées sur cette séquence : ORF4 (position 1403 à position 1957 (protéine de 185 aa)), ORF5 (position 3081 à position 2287 (protéine de 265 aa)), et ORF6 (incomplète ; position 479 à position 1) n'ont pas d'homologues dans les banques de séquences. L'organisation génomique du fragment BamHl I du virus HVT souche FC126 est telle qu'il existe 3 régions intergéniques qui peuvent être utilisées comme sites d'insertion pour des cassettes d'expression de gènes étrangers :

Une région intergénique (région intergénique 1) existe entre l'ORF UL55 et l'ORF HVT gène 3. Une deuxième région intergénique (région intergénique 2) existe entre l'ORF HVT gène 3 et l'ORF 265 aa. Une troisième région intergénique (région intergénique 3) existe entre l'ORF 265 aa et l'ORF 3 HVT. Ces trois régions sont utilisables pour insérer des cassettes d'expression sans affecter la réplication *in vivo* des virus HVT recombinants ainsi obtenus. Des exemples de constructions de plasmides donneurs pour ces régions intergéniques 1, 2 et 3 sont décrits ci-dessous :

### Exemple 5: Construction du plasmide donneur pour la région intergénique 1

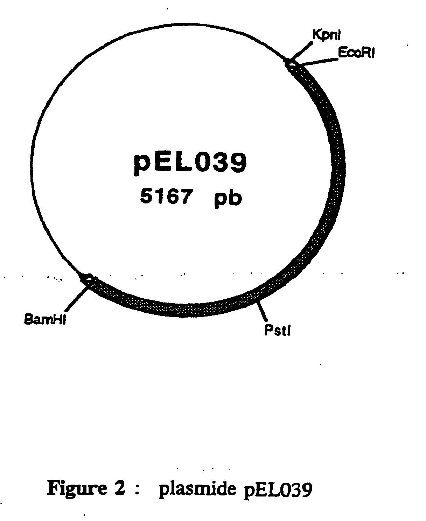

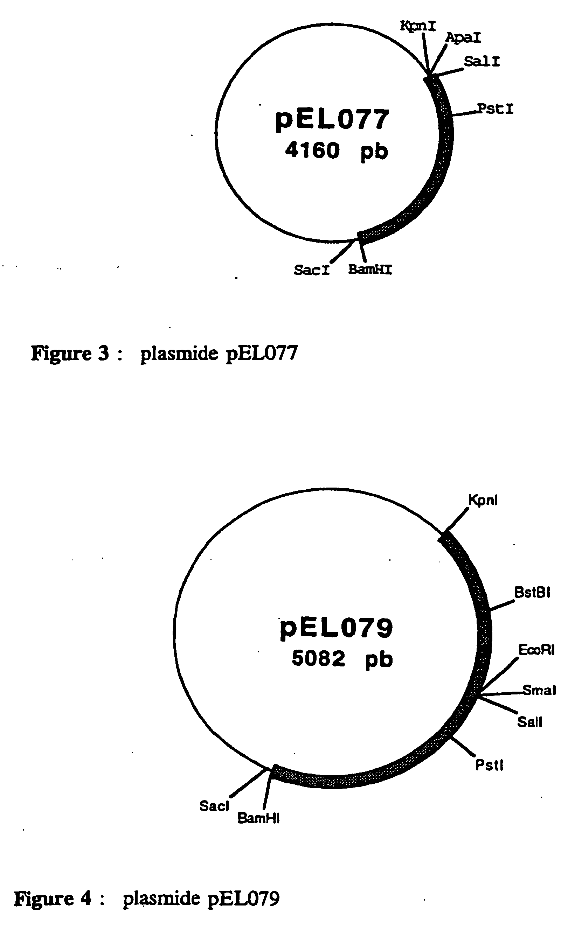

Le plasmide pEL037 a été digéré par BamHl et EcoRI pour isoler les fragments BamHI-EcoRI de 2672 pb et 2163 pb. Ces fragments ont été ligaturés avec le vecteur pBS-SK+, préalablement digéré par BamHl et EcoRI, pour donner respectivement les plasmides pEL039 de 5167 pb et pEL040 de 6104 pb. Le plasmide pEL039 (figure 2) a été digéré par BamHl et Pstl pour isoler le fragment BamHl-Pstl de 997 pb (fragment A). Une PCR a été réalisée avec les oligonucléotides suivants:
EL102 (SEQ ID N° 2) 5' CATTATAAGACCAACGTGCGAGTC 3'
EL161 (SEQ ID N° 3) 5' GTTCACGTCGACAATTATTTTATTTAATAAC 3'
et la matrice pEL039 pour produire un fragment de 420 pb. Ce fragment a été digéré par Pstl et Sall pour isoler un fragment Pstl-Sall de 250 pb (fragment B). Les fragments A et B ont été ligaturés ensemble avec le vecteur pBSll-SK + (Stratagene), préalablement digéré par BamHl et Sall, pour donner le plasmide pEL077 de 4160 pb (figure 3). Le plasmide pEL039 a été digéré par BstBl et Scal pour isoler un fragment BstBl-Scal (bout franc) de 475 pb (fragment C). Une PCR a été réalisée avec les oligonucléotides suivants :
EL147 (SEQ ID N° 4) 5' AAGATAATGGGCTCCCGCTGTTC 3'
EL162 (SEQ ID N° 5) 5' TAATTGTCGACCCCGGGGAATTCGTTTAATGTTAGTTTATTC 3'
et la matrice pEL039 pour produire un fragment PCR de 715 pb. Ce fragment a été digéré par BstBI et Sall pour isoler le fragment BstBI-Sall de 465 pb (fragment D). Les fragments C et D ont été ligaturés ensemble avec le plasmide pEL077, préalablement digéré par Apal, réparé avec la polymérase Klenow, et digéré par Sall, pour donner le plasmide pEL079 de 5082 pb (figure 4). Ce plasmide contient un poly-linker EcoRI-Smal-Sall dans le site intergénique 1.

### Exemple 6: Construction du plasmide donneur pour la région intergénique 2

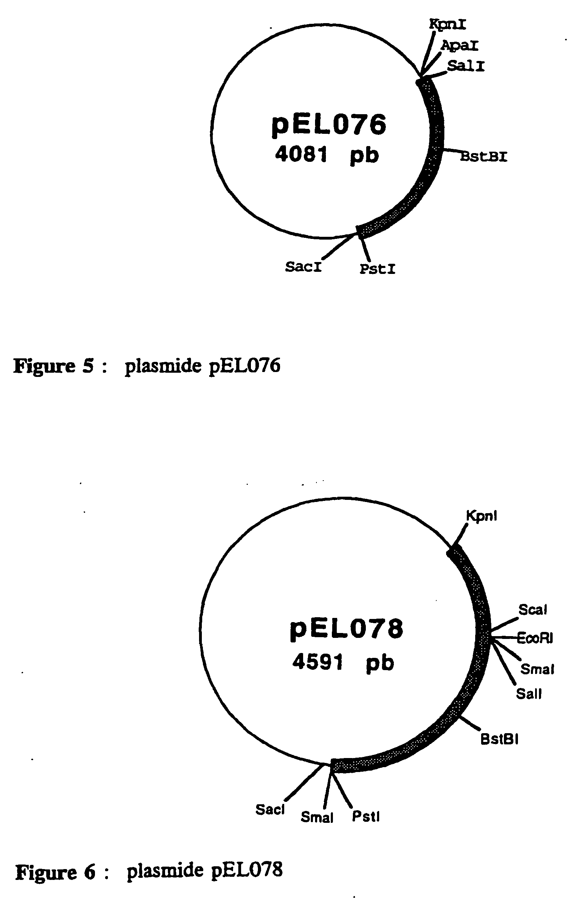

Le plasmide pEL039 (exemple 5) a été digéré par BstBI et Pstl pour isoler le fragment BstBI-PstI de 715 pb (fragment A). Une PCR a été réalisée avec les oligonucléotides suivants:
EL154 (SEQ ID N° 6) 5' GAAATGCAAACTAACATTATTGTC 3'
EL163 (SEQ ID N° 7) 5' GTGTAAATAGTCGACAATATAGATAACGGGC 3'
et la matrice pEL039 pour produire un fragment PCR de 500 pb. Ce fragment a été digéré par BstBl et Sall pour isoler le fragment BstBl-Sall de 430 pb (fragment B). Les fragments A et B ont été ligaturés ensemble avec le vecteur pBSll-SK+, préalablement digéré par Pstl et Sall, pour donner le plasmide pEL076 de 4081 pb (figure 5).

Une autre PCR a été réalisée avec les oligonucléotides suivants:
EL164 (SEQ ID N° 8) 5' CTATATTGTCGACCCCGGGGAATTCATCGACATGATTAAATAC 3'
EL165 (SEQ ID N° 9) 5' CAATGAAGAAATATTTTCTTTGTTCCTTGAAATGC 3'
et la matrice pEL039 pour produire un fragment PCR de 565 pb. Ce fragment a été digéré par Sall et Sspl pour isoler le fragment Sall-Sspl de 535 pb. Ce fragment a été ligaturé avec le plasmide pEL076, préalablement digéré par Apal, réparé avec la polymérase Klenow, et digéré par Sall, pour donner le plasmide pEL078 de 4598 pb (figure 6). Ce plasmide contient un poly-linker EcoRI-Smal-Sall dans la région intergénique 2.

### Exemple 7: Construction du plasmide donneur pour la région intergénique 3

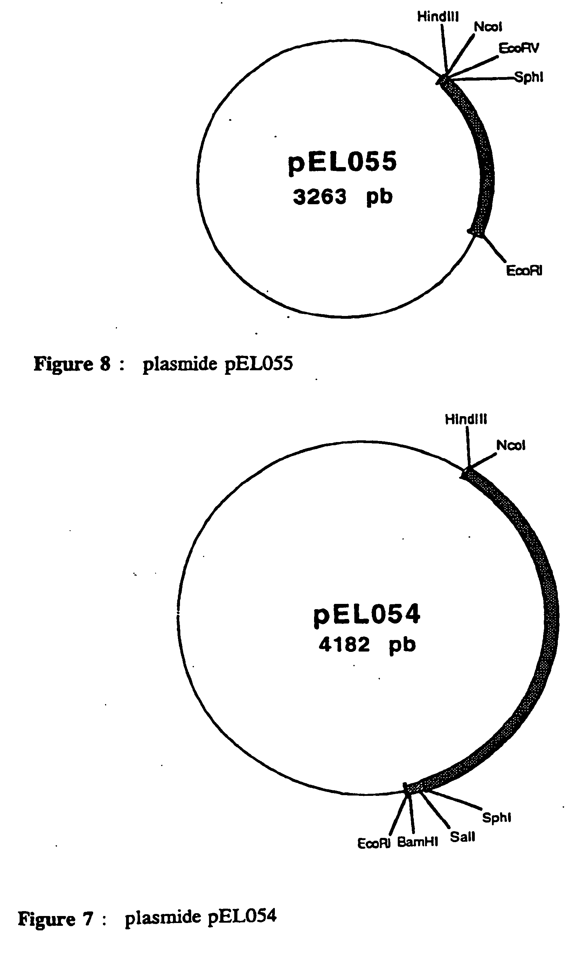

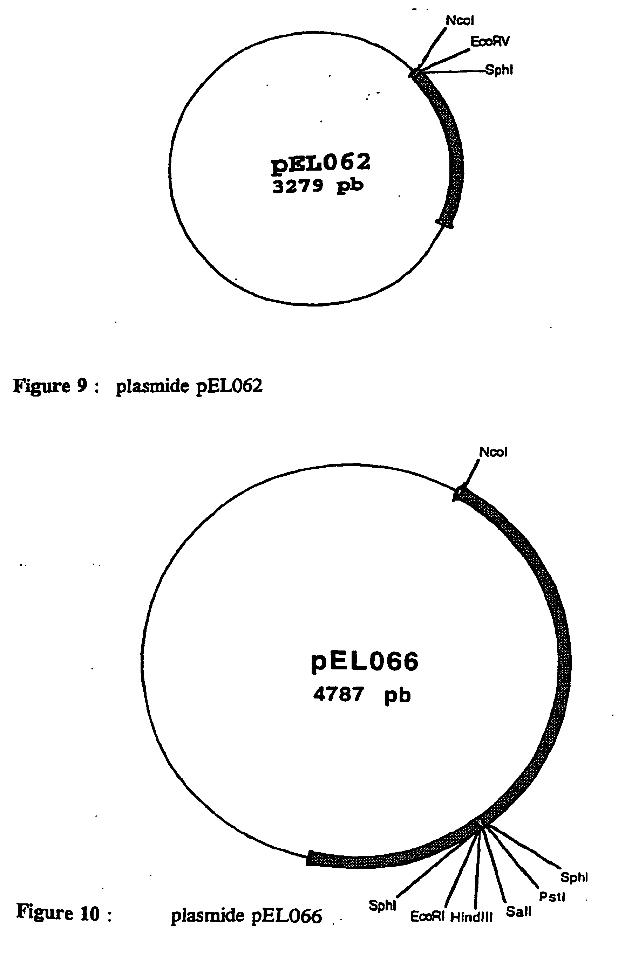

Le plasmide pEL040 (voir exemple 5) a été digéré par Ncol et Sphl pour isoler le fragment Ncol-Sphl de 1468 pb. Ce fragment a été ligaturé avec le plasmide pUC BM20 (Boehringer Mannheim Cat# 1219235), préalablement digéré par Ncol et Sphl, pour donner le plasmide pEL054 de 4182 pb (figure 7). Le plasmide pEL040 a été digéré par EcoRI et Sphl pour isoler le fragment EcoRI-Sphl de 614 pb. Ce fragment a été ligaturé avec le plasmide pUC BM20, préalablement digéré par EcoRI et Sphl, pour donner le plasmide pEL055 de 3263 pb (figure 8). Le plasmide pEL055 a été digéré par EcoRI, réparé par la polymérase Klenow, ligaturé sur lui-même, digéré par Hindlll, réparé avec la polymérase Klenow, et enfin ligaturé sur lui-même pour donner le plasmide pEL062 de 3279 pb (figure 9). Le plasmide pEL054 a été digéré par Ncol et Sall pour isoler le fragment Ncol-Sall de 1492 pb (fragment A). Les deux oligonucléotides suivants:
EL132 (SEQ ID N° 10) 5' CCGAATTCATATAAGCTTACGTG 3'
EL133 (SEQ ID N° 11) 5' TCGACACGTAAGCTTATATGAATTCGGCATG 3'
ont été hybridés entre eux pour produire le fragment Sall-Sphl de 24 pb (fragment B). Les fragments A et B ont été ligaturés ensemble avec le plasmide pEL062, préalablement digéré par Ncol et Sphl, pour donner le plasmide pEL066 de 4787 pb (figure 10). Ce plasmide contient un poly-linker EcoRI-HindIII-Sall dans la région intergénique 3.

### Exemple 8: Construction du plasmide donneur pEL090 et isolement de vHVT16

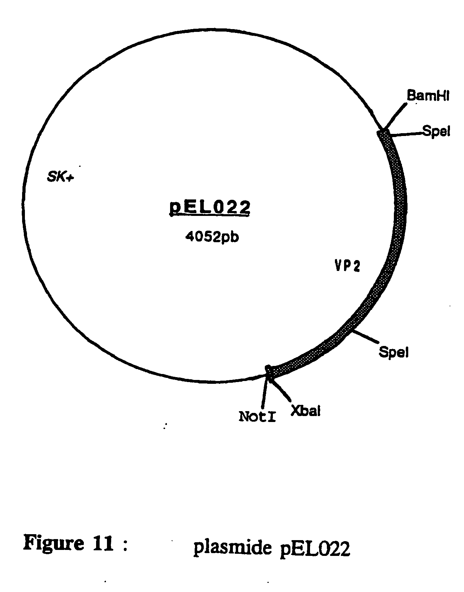

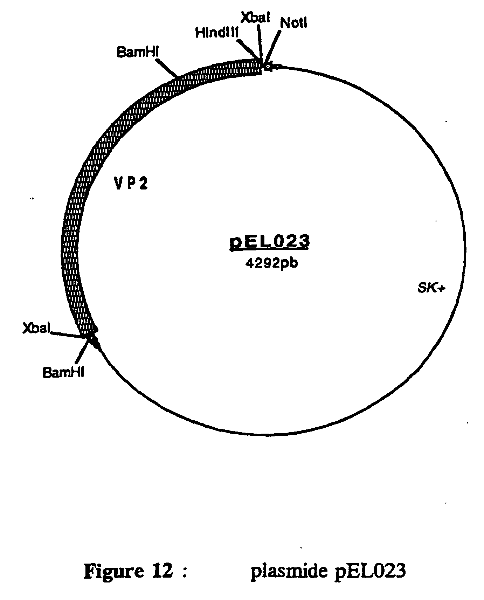

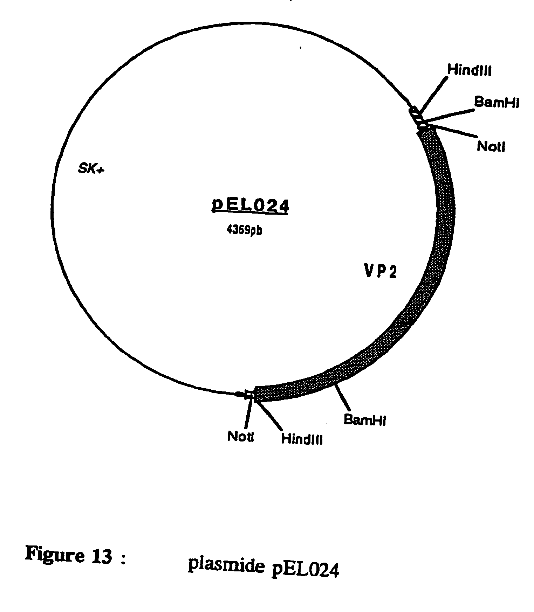

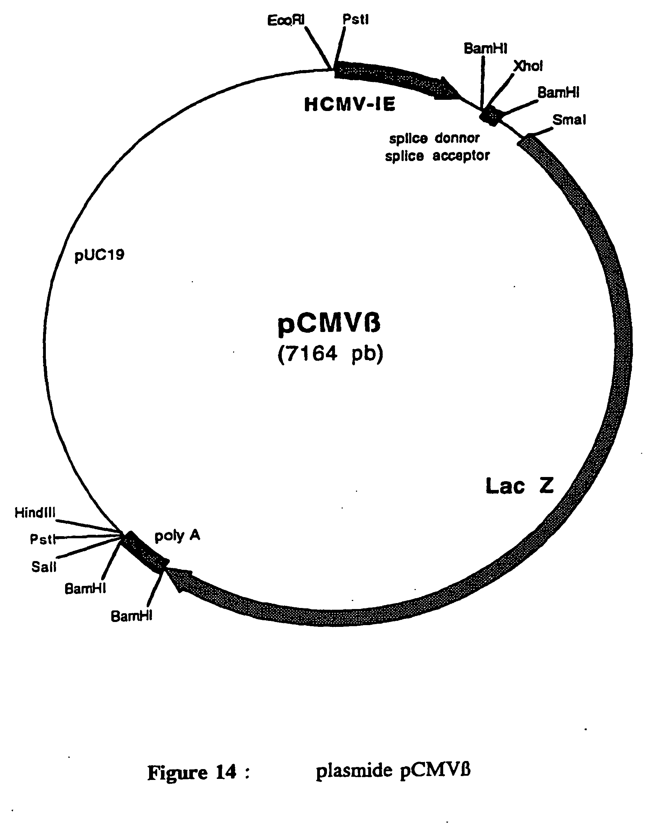

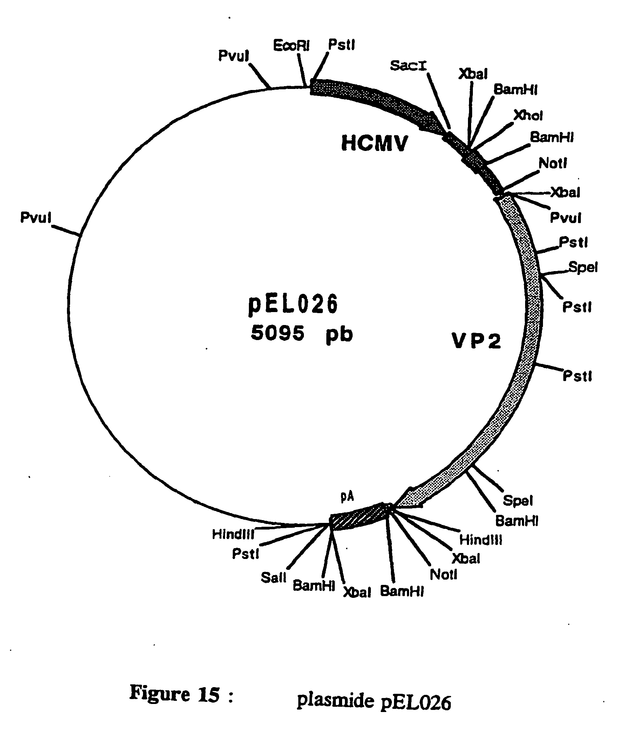

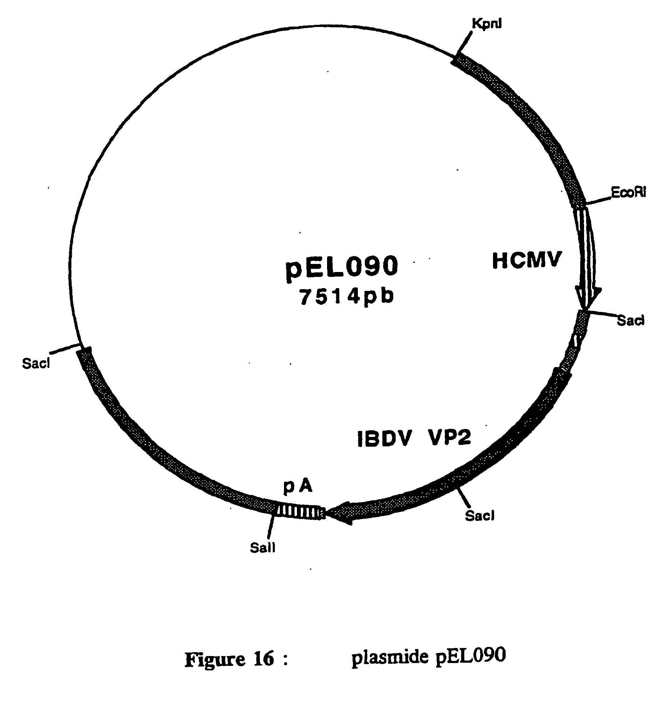

Le plasmide pEL004 (= plasmide pGH004 décrit dans la demande de brevet français 92.13109) contenant le gène IBDV VP2 sous forme d'une cassette BamHl-Hindlll a été digéré par BamHl et Xbal pour isoler le fragment BamHl-Xbal (gène VP2 tronqué) de 1104 pb. Ce fragment a été cloné dans le vecteur pBS-SK +, préalablement digéré avec Xbal et BamHl pour donner le plasmide pEL022 de 4052 pb (figure 11). Le vecteur pBS-SK + a été digéré par EcoRV et Xbal, puis ligaturé sur lui-même pour donner pBS-SK* (modifié). Le plasmide pEL004 a été digéré par Kpnl et Hindlll pour isoler le fragment Kpnl-Hindlll de 1387 pb contenant le gène IBDV VP2 complet. Ce fragment a été cloné dans le vecteur pBS-SK*, préalablement digéré par Kpnl et Hindlll, pour donner le plasmide pEL023 de 4292 pb (figure 12). Le plasmide pEL022 a été digéré par BamHl et Notl pour isoler le fragment BamHl-Notl de 1122 pb (fragment A). Le plasmide pEL023 a été digéré par BamHl et Notl pour isoler le fragment BamHl-Notl de 333 pb (fragment B). Les fragments A et B ont été ligaturés ensemble avec le vecteur pBS-SK +, préalablement digéré par Notl et traité avec la phosphatase alcaline, pour donner le plasmide pEL024 de 4369 pb (figure 13). Le plasmide pEL024 a été digéré par Notl pour isoler le fragment Notl-Notl de 1445 pb. Ce fragment a été ligaturé avec le plasmide pCMVβ (Clontech Cat# 6177-1) (figure 14), préalablement digéré par Notl, pour donner le plasmide pEL026 de 5095 pb (figure 15). Le plasmide pEL026 a été digéré par EcoRI, Sall et Xmnl pour isoler le fragment EcoRI-Sall de 2428 pb. Ce fragment a été ligaturé avec le plasmide pEL079 (voir exemple 5), préalablement digéré par EcoRI et Sall, pour donner le plasmide pEL090 de 7514 pb (figure 16). Ce plasmide permet l'insertion de la cassette d'expression HCMV-IE/IBDV VP2 dans le site intergénique 1 du virus HVT.
Des cellules CEP primaires de 24 heures ont ensuite été transfectées avec le mélange suivant: 1 µg de plasmide pEL090 linéarisé + 5 µg d'ADN viral d'HVT dans 300 µl de milieu OptiMEM (Gibco BRL Cat# 041-01985H) et 100 µg de LipofectAMINE dilués dans 300 µl de milieu (volume final du mélange = 600 µl). Ces 600µl ont ensuite été dilués dans 3 ml (volume final) de milieu et étalés sur 3.10⁶ CEP I. Le mélange a été laissé en contact avec les cellules pendant 5 heures, puis éliminé et remplacé par 5 ml de milieu de culture. Les cellules ont alors été laissées en culture pendant 3 jours à + 37°C, puis elles ont été pronasées, mélangées à des CEP II fraîches (mélange 3:1), et réétalées sur 1 plaque 96 puits. Cette plaque a été laissée en culture pendant 3 jours, puis les cellules ont été pronasées, mélangées à des CEP II fraîches, et réétalées sur 2 plaques de 96 puits, une cupule initiale donnant 2 cupules soeurs. Les plaques 96 puits ont été mises en culture jusqu'à apparition d'un effet cytopathique. Après 72 heures de culture, une des deux plaques 96 puits a été fixée à l'acétone 95% pendant 30 minutes et une réaction d'immunofluorescence indirecte (IFI) a été réalisée avec un anticorps monoclonal anti-VP2 pour rechercher les plages exprimant la protéine VP2. Les cupules "soeurs" des cupules présentant des plages positives en IFI ont été pronasées, mélangées à des CEP II fraîches, et déposées en dilution limite sur des plaques 96 puits. Après 3 jours de culture, les cupules présentant un effet cytopathique ont été pronasées, mélangées à des CEP II, et réétalées sur des plaques 96 puits, une cupule initiale donnant 2 cupules soeurs. 3 jours après, les plages exprimant la protéine VP2 ont été à nouveau recherchées comme précédemment par IFI sur l'une des 2 plaques soeurs.

En général, 4 cycles d'isolement successifs (récolte d'une cupule, réétalement, contrôle par IFI, repiquage d'un cupule soeur...) suffisent pour obtenir des virus recombinants dont la totalité de la progénie présente une fluorescence spécifique. Une plage virale ayant donné 100 % de plages positives en IFI avec un anticorps monoclonal anti-VP2 a été désignée vHVT16. L'ADN génomique de ce virus recombinant a été caractérisé au niveau moléculaire par des techniques classiques de PCR et de Southern blot en utilisant les oligonucléotides et les sondes d'ADN appropriés.

### Exemple 9: Construction du plasmide donneur pEL091 et isolement de vHVT17

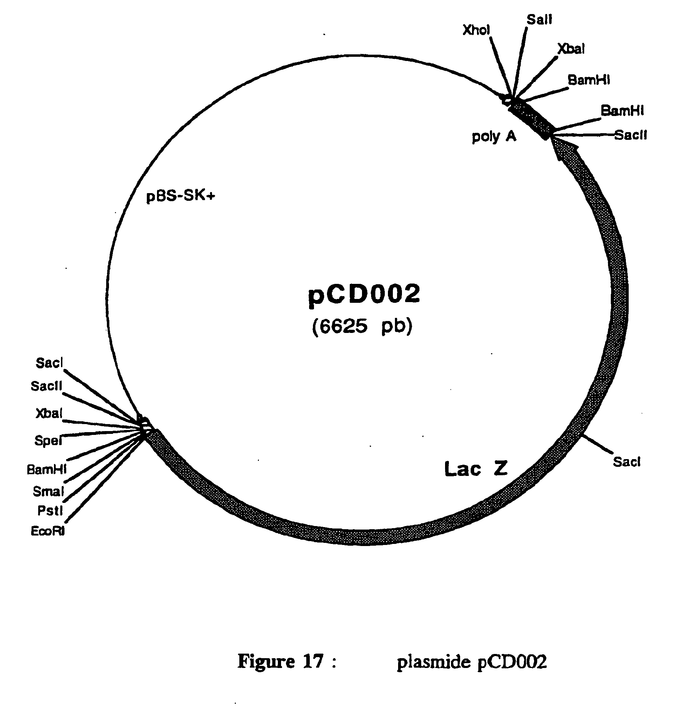

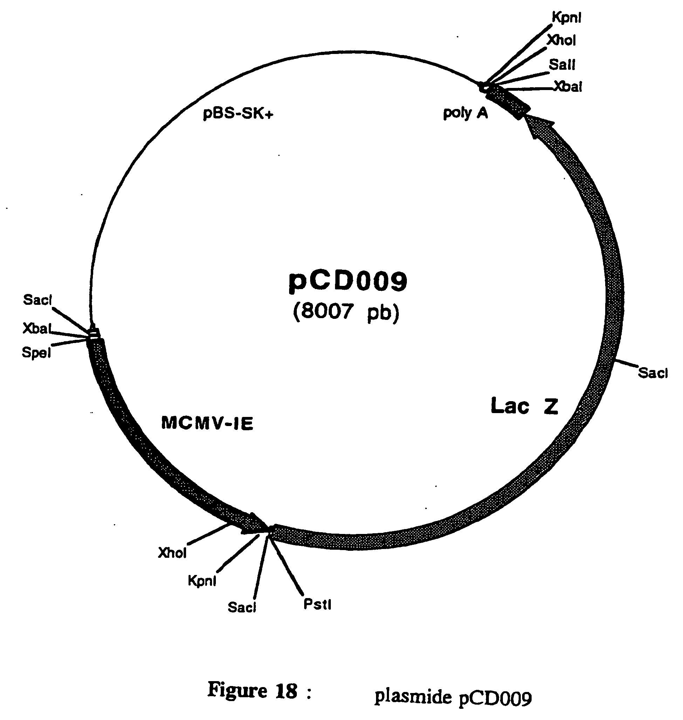

Le plasmide pCMVβ (figure 14) a été digéré par Sall et Smal pour isoler le fragment Sall-Smal de 3679 pb contenant le gène *lacZ* ainsi que le signal de poly-adénylation du gène tardif du virus SV40. Ce fragment a été inséré dans le vecteur pBS-SK +, préalablement digéré par Sall et EcoRV, pour donner le plasmide pCD002 de 6625 pb (figure 17). Ce plasmide contient le gène reporter *lacZ* mais aucun promoteur n'est situé en amont de ce gène. L'ADN génomique viral du virus MCMV a été préparé comme décrit dans l'exemple 2 et digéré par Pstl pour isoler le fragment Pstl-Pstl de 2285 pb. Ce fragment a été cloné dans le vecteur pBS-SK +, préalablement digéré par Pstl et traité avec la phosphatase alcaline, pour donner le plasmide pCD004. Le plasmide pCD004 a été digéré par Hpal et Pstl pour isoler le fragment Hpal-Pstl de 1389 pb qui contient la région promotrice/activatrice du gène Immediate-Early du cytomégalovirus murin (Murine CytoMegaloVirus = MCMV) (Dorsch-Häsler K. et al. Proc. Natl. Acad. Sci. 1985. 82. 8325-8329, et demande de brevet WO-A-87/03905). Ce fragment a été cloné dans le plasmide pCD002 préalablement digéré par Pstl et Smal, pour donner le plasmide pCD009 de 8007 pb (figure 18).

Un oligonucléotide double brin a été obtenu par hybridation des deux oligonucléotides suivants :
MB070 (SEQ ID N° 12) 5' CGAATTCACTAGTGTGTGTCTGCAGGCGGCCGCGTGTGTGTCGACGGTAC 3'
MB071 (SEQ ID N° 13) 5' CGTCGACACACACGCGGCCGCCTGCAGACACACACTAGTGAATTCGAGCT 3'

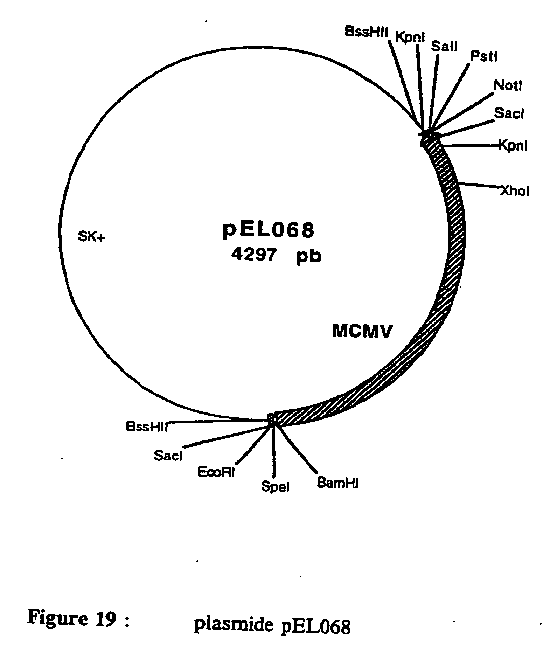

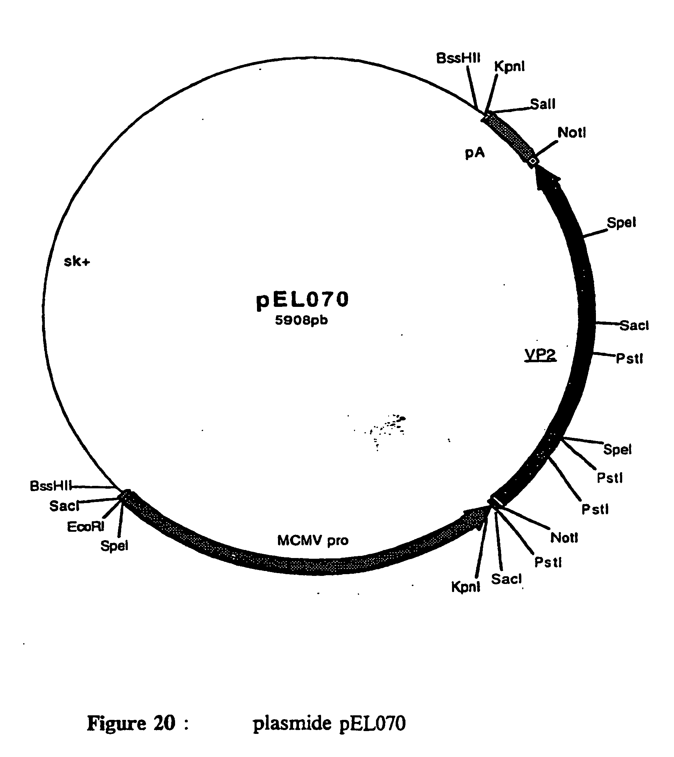

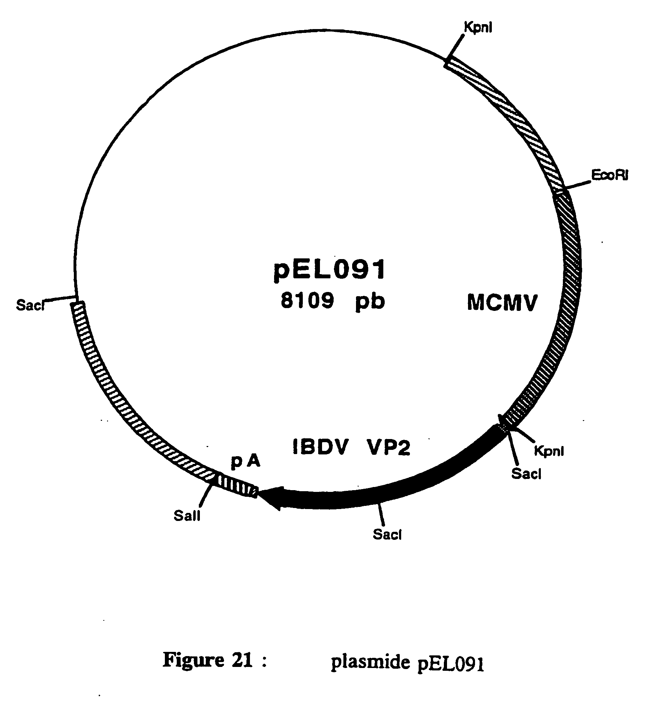

Cet oligonucléotide double brin a été ligaturé dans le vecteur pBS-SK+, préalablement digéré par Kpnl et Sacl, pour donner le plasmide pEL067. Le plasmide pCD009 a été digéré par Pstl et Spel pour isoler le fragment Pstl-Spel de 1396 pb. Ce fragment a été ligaturé avec le plasmide pEL067, préalablement digéré par Pstl et Spel, pour donner le plasmide pEL068 de 4297 pb (figure 19). Le plasmide pEL026 (voir exemple 8) a été digéré par Hindlll et Sall pour isoler le fragment Hindlll-Sall de 235 pb (fragment B). Les fragments A et B ont été ligaturés ensemble avec le plasmide pEL068, préalablement digéré par Notl et Sall, pour donner le plasmide pEL070 de 5908 pb (figure 20). Le plasmide pEL070 a été digéré par EcoRI, Sall et Xmnl pour isoler le fragment EcoRI-SalI, de 3035 pb. Ce fragment a été ligaturé avec la plasmide pELO79 (voir exemple 5), préalablement digéré par EcoRI et Sall, pour donner le plasmide pEL091 de 8109 pb (figure 21). Ce plasmide permet l'insertion de la cassette d'expression MCMV-IE/IBDV VP2 dans le site intergénique 1 du virus HVT.

Une co-transfection réalisée, comme décrit dans l'exemple 8, avec le plasmide pEL091 et l'ADN génomique du virus HVT a conduit à l'isolement et à la purification du recombinant vHVT17.

### Exemple 10: Construction du plasmide donneur pEL092 et isolement de vHVT18

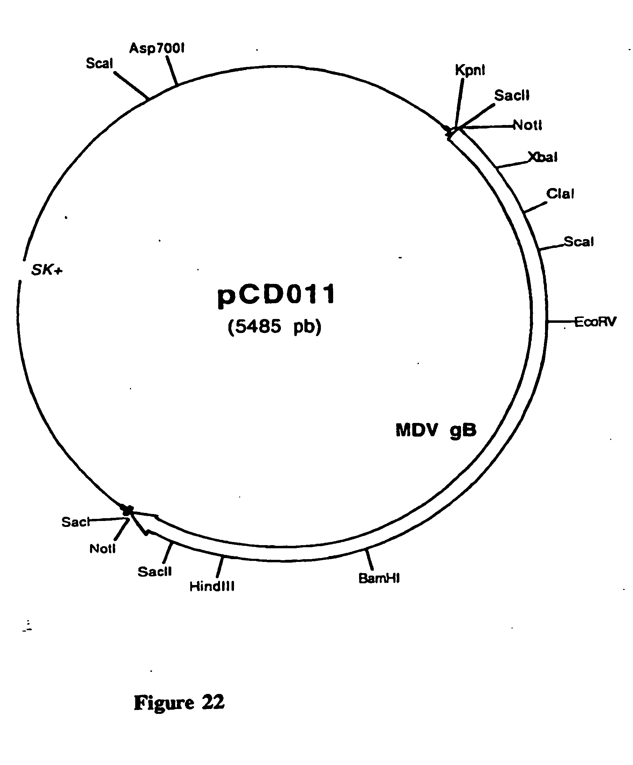

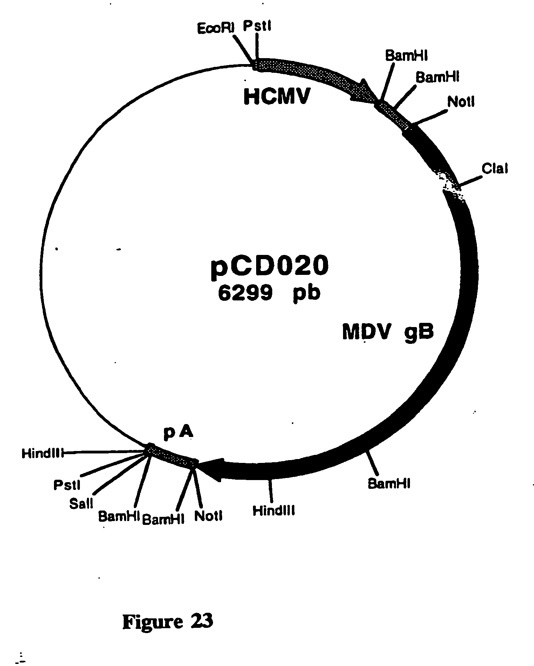

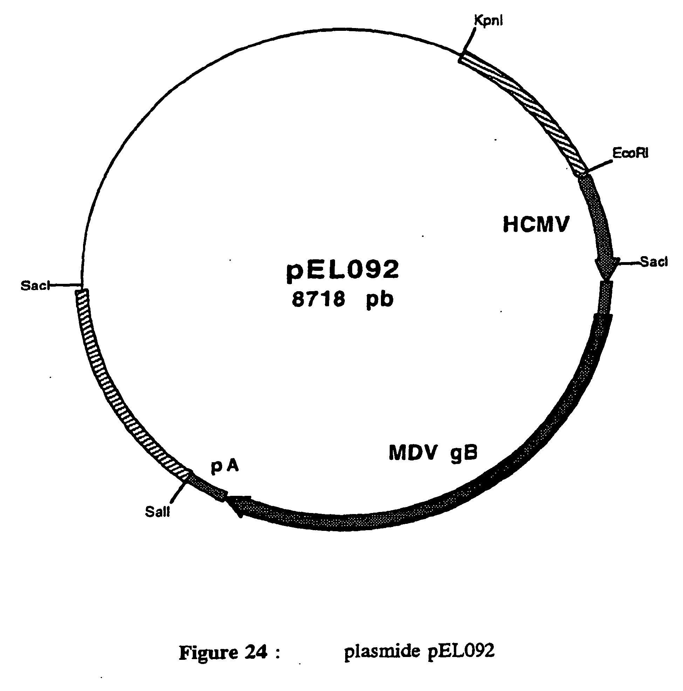

Le fragment EcoRI-Sall de 3,9 kpb de l'ADN génomique du virus MDV souche RB1 B contenant le gène MDV gB (séquence publiée par Ross N. et al. J. Gen. Virol. 1989. 70. 1789-1804) a été ligaturé avec le vecteur pUC13, préalablement digéré par EcoRI et Sall, pour donner le plasmide pCD007. Ce plasmide a été digéré par Sacl et Xhol pour isoler le fragment Sacl-Xhol de 2260 pb (partie centrale du gène gB = fragment A). Une PCR a été réalisée avec les oligonucléotides suivants:
CD001 (SEQ ID N° 14) 5' GACTGGTACCGCGGCCGCATGCACTTTTTAGGCGGAATTG 3'
CD002 (SEQ ID N° 15) 5' TTCGGGACATTTTCGCGG 3'
et la matrice pCD007 pour produire un fragment PCR de 222 pb. Ce fragment a été digéré par Kpnl et Xbal pour isoler un fragment Kpnl-Xbal de 190 pb (extrémité 5' du gène gB = fragment B). Une autre PCR a été réalisée avec les oligonucléotides suivants:
CD003 (SEQ ID N° 16) 5' TATATGGCGTTAGTCTCC 3'
CD004 (SEQ ID N° 17) 5' TTGCGAGCTCGCGGCCGCTTATTACACAGCATCATCTTCTG 3'
et la matrice pCD007 pour produire un fragment PCR de 195 pb. Ce fragment a été digéré par Sacl et Sacll pour isoler le fragment Sacl-Sacll de 162 pb (extrémité 3' du gène gB = fragment C). Les fragments A, B et C ont été ligaturés ensemble avec le vecteur pBS-SK +, préalablement digéré par Kpnl et Sacl, pour donner le plasmide pCD011 de 5485 pb (figure 22). Le plasmide pCD011 a été digéré par Notl pour isoler le fragment Notl-Notl de 2608 pb (gène MDV gB entier). Ce fragment a été ligaturé avec le plasmide pCMVβ, préalablement digéré par Notl et traité avec la phosphatase alcaline, pour donner le plasmide pCD020 de 6299 pb (figure 23) (Dans ce plasmide, le gène MDV gB remplace le gène *lacZ*). Le plasmide pCD020 a été digéré par EcoRI et Sall pour isoler le fragment EcoRI-Sall de 3648 pb. Ce fragment a été ligaturé avec le plasmide pEL079 (voir exemple 5), préalablement digéré par EcoRI et Sall, pour donner le plasmide pEL092 de 8718 pb (figure 24). Ce plasmide permet l'insertion de la cassette d'expression HCMV-IE/MDV gB dans le site intergénique 1 du virus HVT.

Une co-transfection réalisée, comme décrit dans l'exemple 8, avec le plasmide pEL092 et l'ADN génomique du virus HVT a conduit à l'isolement et à la purification du recombinant vHVT18.

### Exemple 11: Construction du plasmide donneur pEL093 et isolement de vHVT19

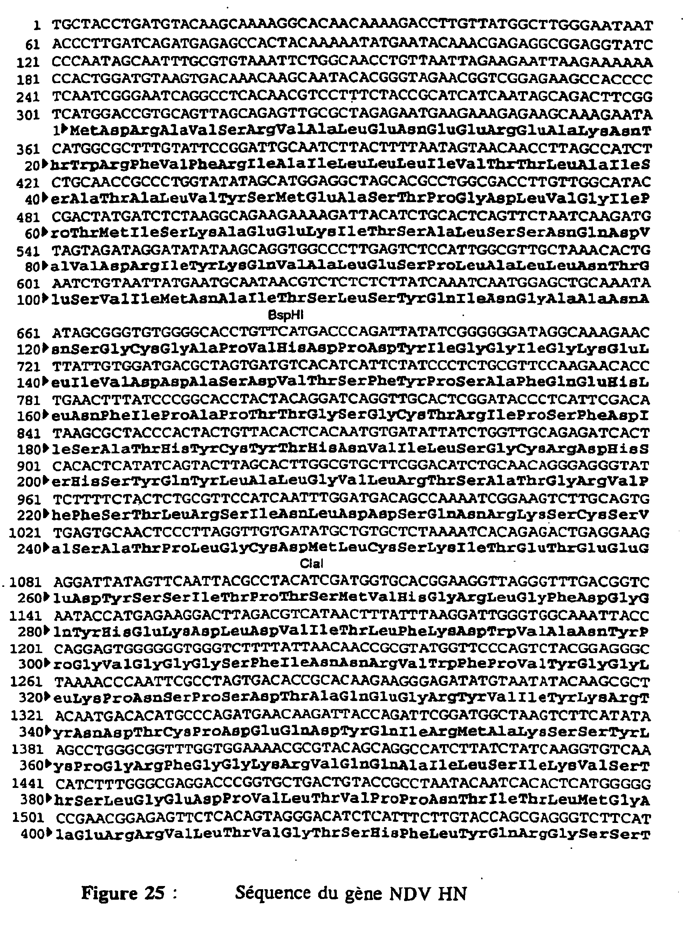

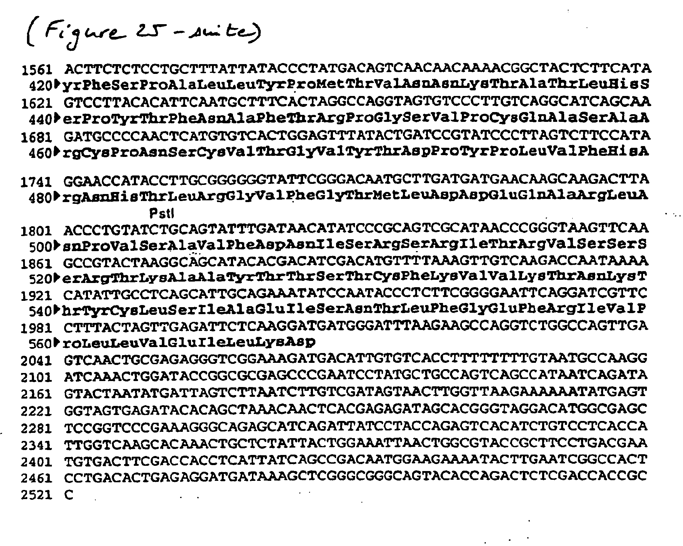

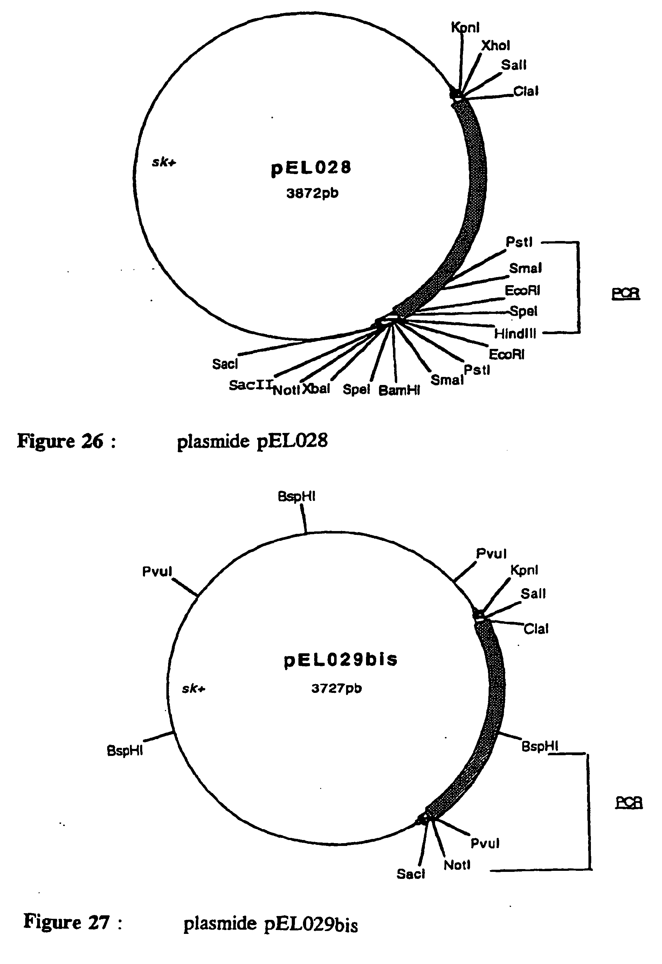

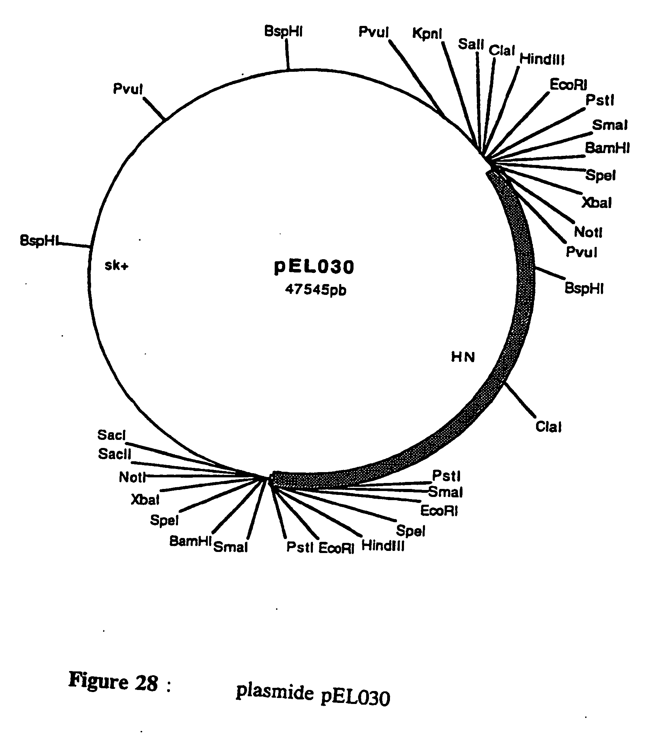

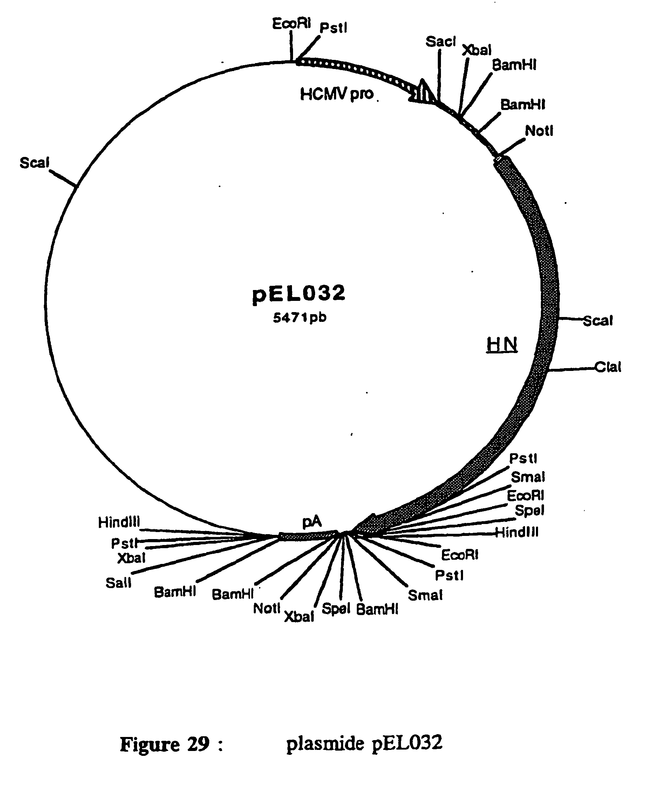

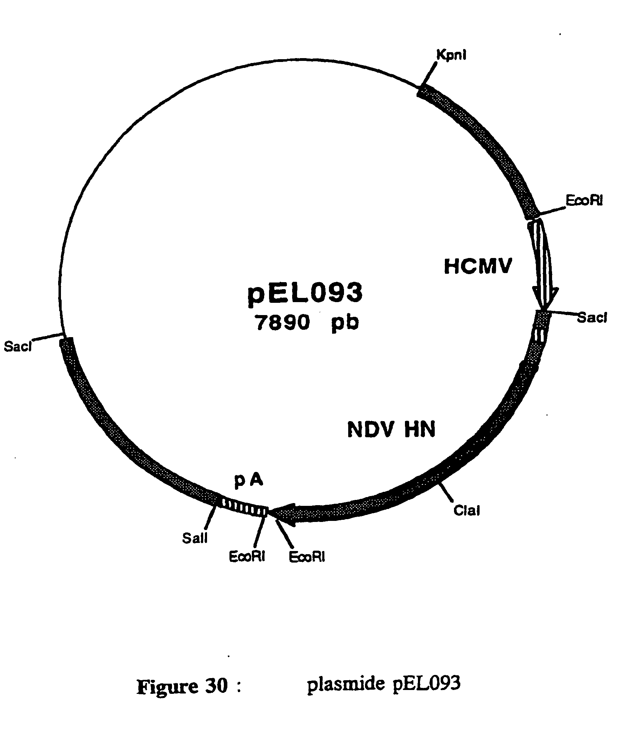

La constitution d'une banque d'ADN complémentaire du génome du virus de la maladie de Newcastle (NDV), souche Texas, a été réalisée comme décrit par Taylor J. et al. (J. Virol. 1990. 64. 1441-1450). Un clone pBR322 contenant la fin du gène fusion (F), la totalité du gène hémagglutinine-neuraminidase (HN) et le début du gène de la polymérase a été identifié pHN01. La séquence du gène NDV HN présent sur ce clone est présentée sur la figure 25 (SEQ ID N° 18). Le plasmide pHN01 a été digéré par Sphl et Xbal pour isoler le fragment Sphl-Xbal de 2520 pb. Ce fragment a été ligaturé avec le vecteur pUC19, préalablement digéré par Sphl et Xbal, pour donner le plasmide pHN02 de 5192 pb. Le plasmide pHN02 a été digéré par Clal et Pstl pour isoler le fragment Clal-Pstl de 700 pb (fragment A). Une PCR a été réalisée avec les oligonucléotides suivants:
EL071 (SEQ ID N° 19) 5' CAGACCAAGCTTCTTAAATCCC 3'
EL073 (SEQ ID N° 20) 5' GTATTCGGGACAATGC 3'
et la matrice pHN02 pour produire un fragment PCR de 270 pb. Ce fragment a été digéré par Hindlll et Pstl pour isoler un fragment Hindlll-Pstl de 220 pb (fragment B). Les fragments A et B ont été ligaturés ensemble avec le vecteurs pBS-SK+, préalablement digéré par Clal et Hindlll, pour donner le plasmide pEL028 de 3872 pb (figure 26). Le plasmide pHN02 a été digéré par Bsphl et Clal pour isoler le fragment Bsphl-Clal de 425 pb (fragment C). Une PCR a été réalisée avec les oligonucléotides suivants:
EL074 (SEQ ID N° 21) 5' GTGACATCACTAGCGTCATCC 3'
EL075 (SEQ ID N° 22) 5' CCGCATCATCAGCGGCCGCGATCGGTCATGGACAGT 3'
et la matrice pHN02 pour produire un fragment PCR de 425 pb. Ce fragment a été digéré par Bsphl et Notl pour isoler le fragment Bsphl-Notl de 390 pb (fragment D). Les fragments C et D ont été ligaturés ensemble avec le vecteur pBS-SK +, préalablement digéré par Clal et Notl, pour donner le plasmide pEL029bis de 3727 pb (figure 27). Le plasmide pEL028 a été digéré par Clal et Sacll pour isoler le fragment Clal-Sacll de 960 pb (fragment E). Le plasmide pEL029bis a été digéré par Clal et Notl pour isoler le fragment Clal-Notl de 820 pb (fragment F). Les fragments E et F ont été ligaturés ensemble avec le vecteur pBS-SK+, préalablement digéré par Notl et Sacll, pour donner le plasmide pEL030 de 4745 pb (figure 28). Le plasmide pEL030 a été digéré par Notl pour isoler le fragment Notl-Notl de 1780 pb (gène NDV HN entier). Ce fragment a été ligaturé, à la place du gène *lacZ,* avec le plasmide pCMVβ, préalablement digéré par Notl et traité avec la phophatase alcaline, pour donner le plasmide pEL032 de 5471 pb (figure 29). Le plasmide pEL032 a été digéré par EcoRI et Clal pour isoler le fragment EcoRI-Clal de 1636 pb (fragment G). Le plasmide pEL032 a été digéré par Clal et Sall pour isoler le fragment Clal-Sall de 1182 pb (fragment H). Les fragments G et H ont été ligaturés ensemble avec le plasmide pEL079 (voir exemple 5), préalablement digéré par EcoRI et Sall, pour donner le plasmide pEL093 de 7890 pb (figure 30). Ce plasmide permet l'insertion de la cassette d'expression HCMV-IE/NDV HN dans le site intergénique 1 du virus HVT.

Une co-transfection réalisée, comme décrit dans l'exemple 8, avec le plasmide pEL093 et l'ADN génomique du virus HVT a conduit à l'isolement et à la purification du recombinant vHVT19.

### Exemple 12: Construction du plasmide donneur pEL094 et isolement de vHVT20

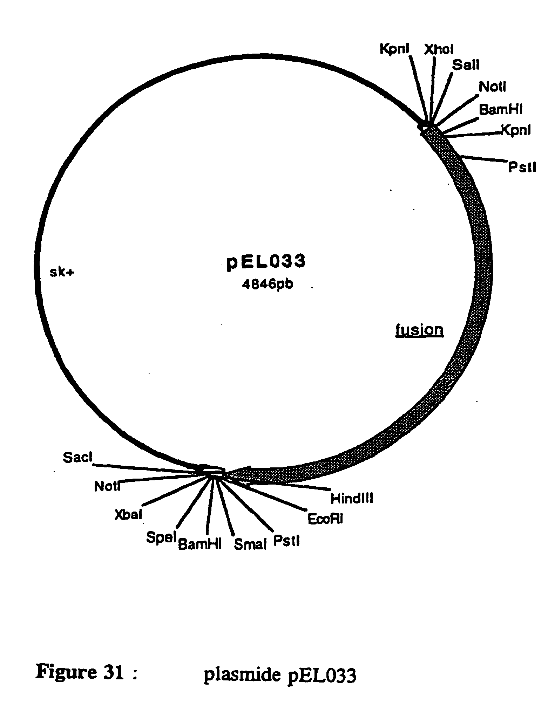

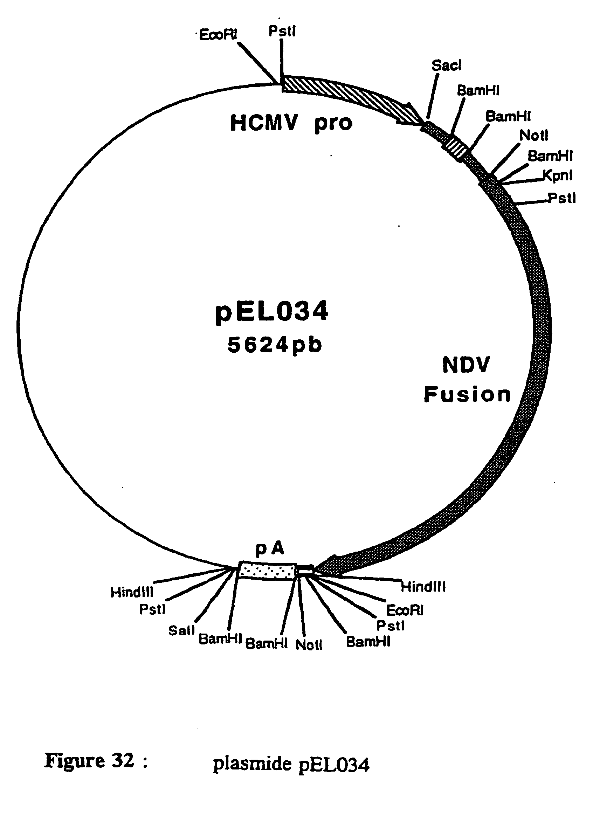

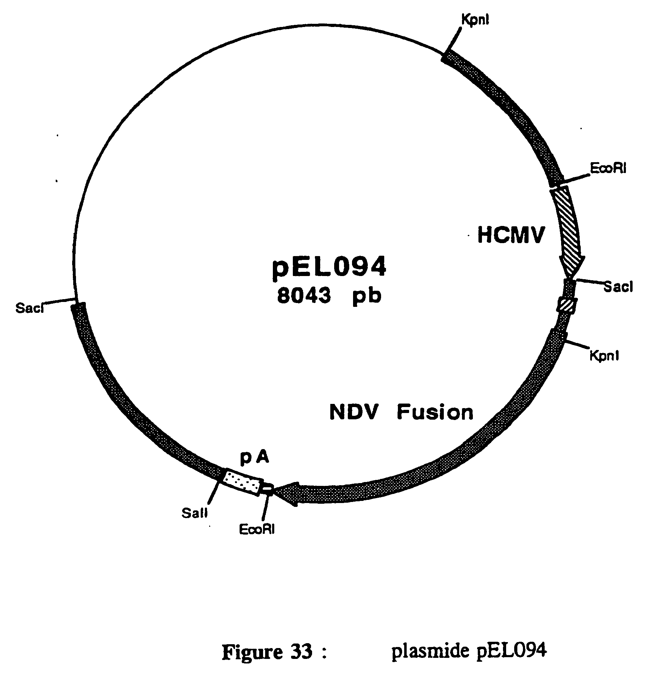

Un clone provenant de la banque d'ADN complémentaire du génome du virus de la maladie de Newcastle (voir exemple 11) et contenant le gène fusion (F) en entier a été appelé pNDV81. Ce plasmide a été décrit précédemment et la séquence du gène NDV F présent sur ce clone a été publiée (Taylor J. et al. J. Virol. 1990. 64. 1441-1450). Le plasmide pNDV81 a été digéré par Narl et Pstl pour isoler le fragment Narl-Pstl de 1870 pb (fragment A). Une PCR a été réalisée avec les oligonucléotides suivants:
EL076 (SEQ ID N° 23) 5' TGACCCTGTCTGGGATGA 3'
EL077 (SEQ ID N° 24) 5'GGATCCCGGTCGACACATTGCGGCCGCAAGATGGGC 3'
et la matrice pNDV81 pour produire un fragment de 160 pb. Ce fragment a été digéré par Pstl et Sall pour isoler le fragment Pstl-Sall de 130 pb (fragment B). Les fragments A et B ont été ligaturés ensemble avec le vecteur pBS-SK+, préalablement digéré par Clal et Sall, pour donner le plasmide pEL033 de 4846 pb (figure 31). Le plasmide pEL033 a été digéré par Notl pour isoler le fragment Notl-Notl de 1935 pb (gène F entier). Ce fragment a été ligaturé avec le plasmide pCMVβ, préalablement digéré par Notl et traité avec la phosphatase alcaline, pour donner le plasmide pEL034 de 5624 pb (le gène NDV F a remplacé le gène *lacZ*) (figure 32). Le plasmide pEL034 a été digéré par EcoRI et Kpnl pour isoler le fragment EcoRI-Kpnl de 866 pb (fragment C). Le plasmide pEL034 a été digéré par Kpnl et Sall pour isoler le fragment Kpnl-Sall de 2114 pb (fragment D). Les fragments C et D ont été ligaturés ensemble avec le plasmide pEL079 (voir exemple 5), préalablement digéré par EcoRI et Sall, pour donner le plasmide pEL094 de 8043 pb (figure 33). Ce plasmide permet l'insertion de la cassette d'expression HCMV-IE/NDV F dans le site intergénique 1 du virus HVT.

Une co-transfection réalisée, comme décrit dans l'exemple 8, avec le plasmide pEL094 et l'ADN génomique du virus HVT a conduit à l'isolement et à la purification du recombinant vHVT20.

### Exemple 13: Construction du plasmide donneur pEL095 et isolement de vHVT21

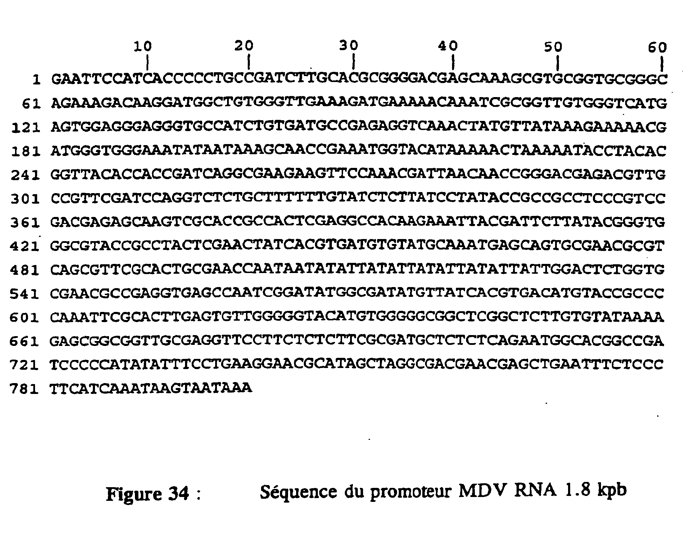

Les séquences situées en amont du gène MDV RNA 1.8 kpb sont décrites dans Bradley G. *et al.* (J. Virol. 1989. **63.** 2534-2542) (figure 34 et SEQ ID N° 25). Une amplification PCR a été réalisée à partir d'ADN extrait de lymphocytes récoltés sur des poulets infectés par la souche MDV RB1B (voir exemple 1) avec les oligonucléotides suivants:
MBO47 (SEQ ID N° 26) 5' GGTCTACTAGTATTGGACTCTGGTGCGAACGC 3'
MBO48 (SEQ ID N° 27) 5' GTCCAGAATTCGCGAAGAGAGAAGGAACCTC 3'

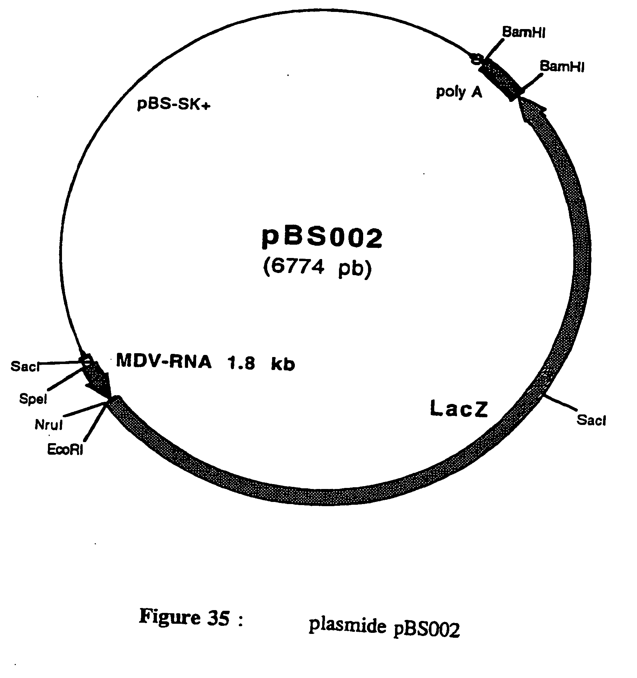

Le fragment PCR de 163 pb ainsi obtenu a été digéré par EcoRI et Spel, puis ligaturé avec le plasmide pCD002 (voir exemple 9), préalablement digéré par EcoRI et Spel, pour donner le plasmide pBS002 de 6774 pb (figure 35). Le plasmide pBS002 contient le promoteur du gène MDV RNA 1.8 kb cloné en amont du gène *lacZ.*

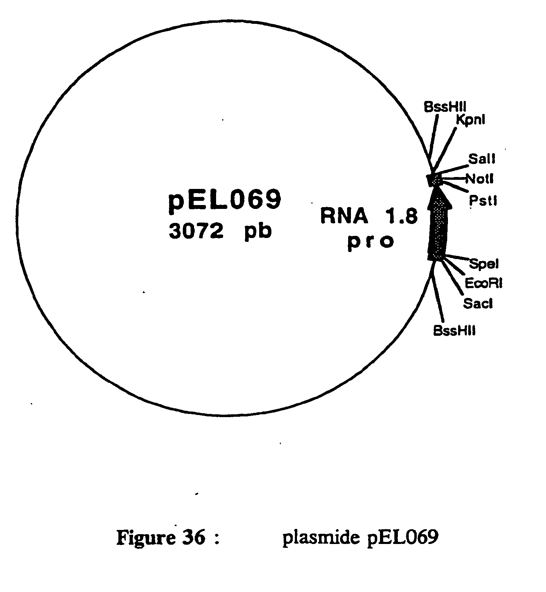

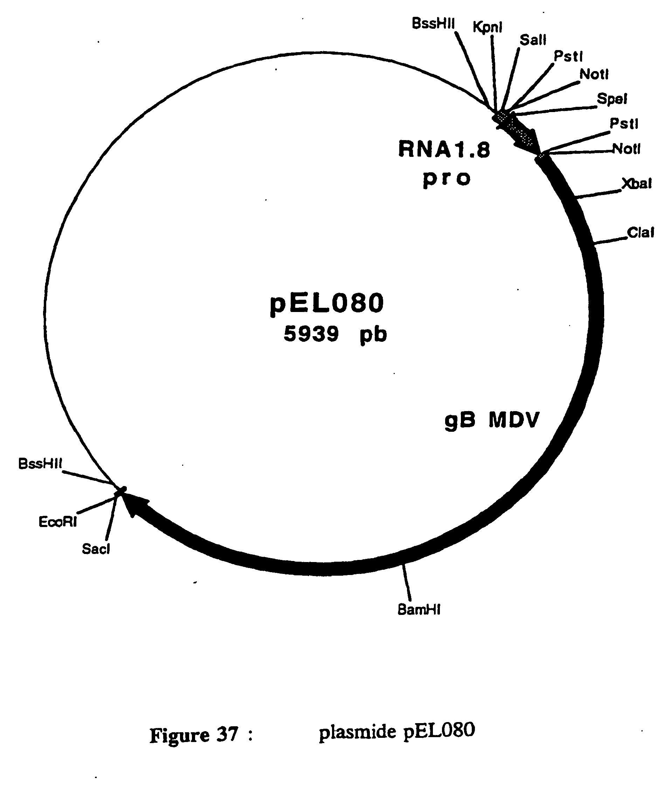

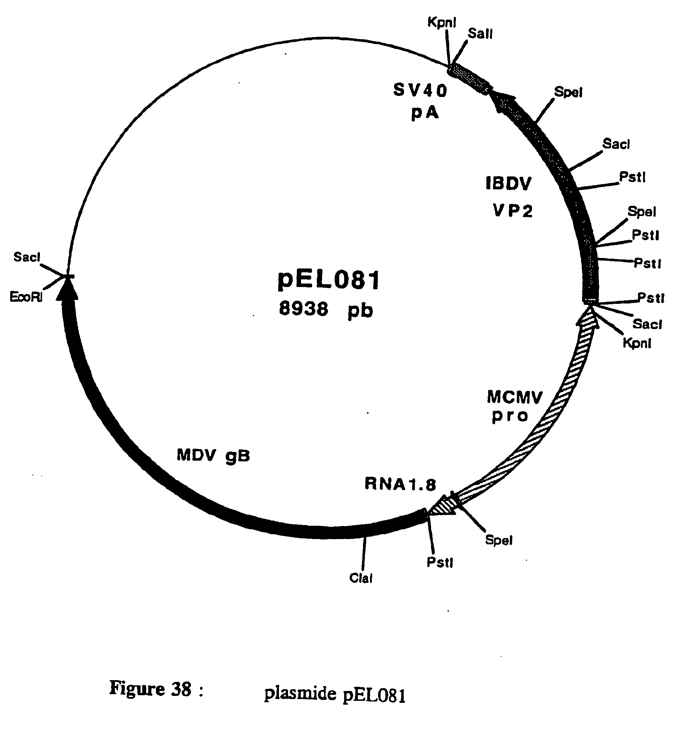

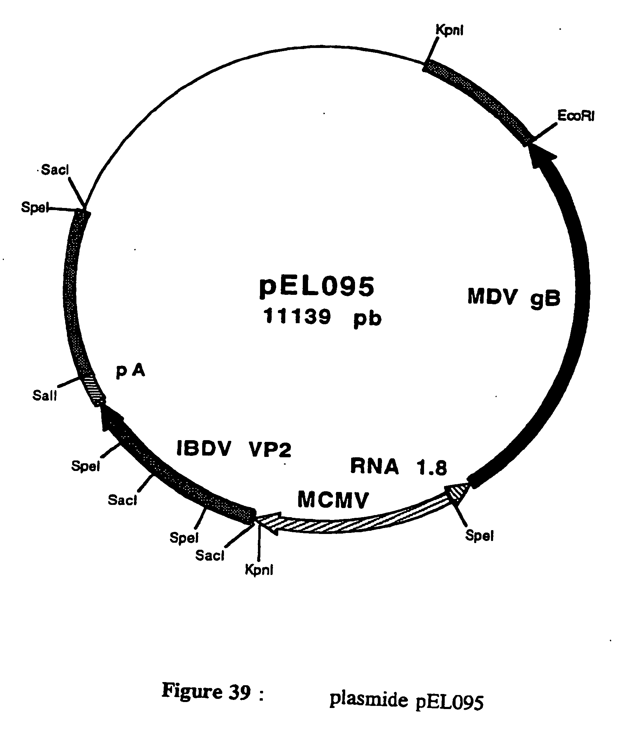

Une PCR a été réalisée avec les oligonucléotides:
MB047 (SEQ ID N° 26) et
MB072 (SEQ ID N° 28) 5' GTGTCCTGCAGTCGCGAAGAGAGAAGGAACCTC 3'
et la matrice pBS002. Le fragment PCR ainsi obtenu a été digéré par Pstl et Spel pour isoler un fragment Pstl-Spel de 200 pb. Ce fragment a été ligaturé avec le plasmide pEL067 (voir exemple 9), préalablement digéré par Pstl et Spel, pour donner le plasmide pEL069 (figure 36). Le plasmide pCD007 (voir exemple 10) a été digéré par EcoRI et Xbal pour isoler le fragment EcoRl-Xbal de 2670 pb (fragment A). Le plasmide pCD011 (voir exemple 10) a été digéré par Notl et Xbal pour isoler le fragment Notl-Xbal de 180 pb (fragment B). Le plasmide pEL069 a été digéré par Notl et Spel pour isoler le fragment Notl-Spel de 180 pb (fragment C). Les fragments A, B et C ont été ligaturés ensemble avec le plasmide pEL067 (voir exemple 9), préalablement digéré par EcoRI et Spel, pour donner le plasmide pEL080 de 5939 pb (figure 37). Le plasmide pEL070 (voir exemple 9) a été digéré par Kpnl et Spel pour isoler le fragment Kpnl-Spel de 1345 pb (fragment D). Le plasmide pEL070 a aussi été digéré par Kpnl et Sall pour isoler le fragment Kpnl-Sall de 1658 pb (fragment E). Les fragments D et E ont été ligaturés ensemble avec le plasmide pEL080, préalablement digéré par Sall et Spel, pour donner le plasmide pEL081 de 8938 pb (figure 38). Le plasmide pEL081 a été digéré par EcoRI et Sall pour isoler le fragment EcoRI-Sall de 6066 pb. Ce fragment a été ligaturé avec le plasmide pEL079 (voir exemple 5), préalablement digéré par EcoRI et Sall, pour donner finalement le plasmide pEL095 de 11139 pb (figure 39). Ce plasmide permet d'insérer la double cassette d'expression VP2/MCMV-IE//RNA 1.8 kpb/MDV gB dans le site intergénique 1 du virus HVT.

Une co-transfection réalisée, comme décrit dans l'exemple 8, avec le plasmide pEL095 et l'ADN génomique du virus HVT a conduit à l'isolement et à la purification du recombinant vHVT21.

### Exemple 14: Construction du plasmide donneur pEL098 et isolement de vHVT24

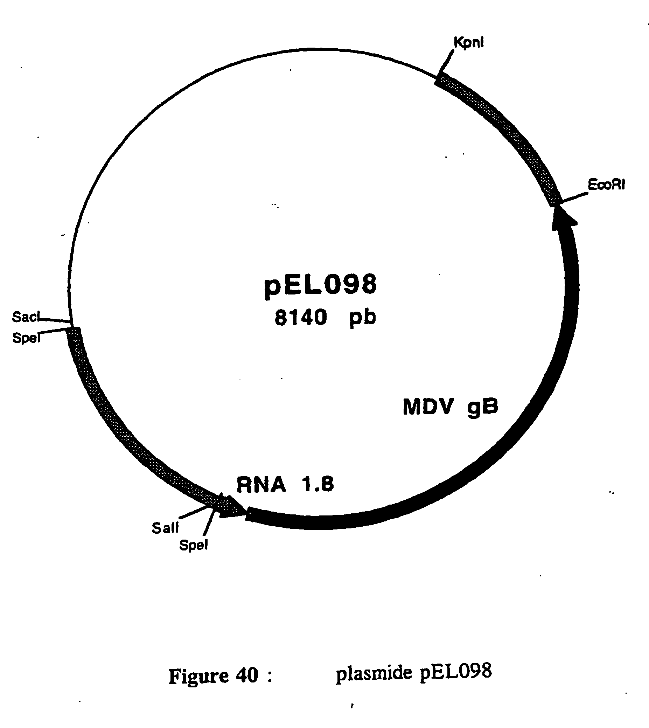

Le plasmide pEL080 (voir exemple 13) a été digéré par EcoRI et Sall pour isoler le fragment EcoRI-Sall de 3040 pb (cassette RNA 1.8 kpb / MDV gB). Ce fragment a été ligaturé avec le plasmide pEL079 (voir exemple 5), préalablement digéré par EcoRI et Sall, pour donner le plasmide pEL098 de 8140 pb (figure 40). Ce plasmide permet l'insertion de la cassette RNA 1.8 kpb / MDV gB dans le site intergénique 1 du virus HVT.

Une co-transfection réalisée, comme décrit dans l'exemple 8, avec le plasmide pEL098 et l'ADN génomique du virus HVT a conduit à l'isolement et à la purification du recombinant vHVT24.

### Exemple 15: Construction de plasmides donneurs pour l'insertion de cassettes d'expression IBV M et S dans le site intergénique 1 du virus HVT

Selon la même stratégie que celle décrite plus haut pour l'insertion de cassettes d'expression (gènes placés sous le contrôle des promoteurs HCMV-IE ou MCMV-IE ou double promoteur MCMV-IE//RNA 1.8 kpb) dans le site intergénique 1, il est possible de réaliser des virus HVT recombinants exprimant à un niveau élevé les protéines membrane (M) ou spike (S) du virus de la bronchite infectieuse aviaire (IBV). On réalise de préférence une construction où le gène IBV S est sous la dépendance du promoteur HCMV-IE ou du promoteur MCMV-IE, ou bien une construction où les gènes IBV M et IBV S sont insérés ensemble avec le double promoteur MCMV-IE/RNA 1.8 kpb dans le site intergénique 1, le gène M étant sous le contrôle du promoteur RNA 1.8 kpb et le gène S étant sous le contrôle du promoteur MCMV-IE. Dans cette configuration, le promoteur RNA 1.8 kpb est activé par la région activatrice du promoteur MCMV-IE.

### Exemple 16: Construction de virus HVT recombinants comprenant des gènes étrangers insérés dans les sites intergéniques 2 et 3

L'obtention de virus HVT recombinants ayant inséré des cassettes d'expression de gènes étrangers dans les sites intergéniques 2 et 3 se fait en suivant la stratégie décrite pour les exemples 8 à 14, mais en utilisant respectivement les plasmides pEL078 (site intergénique 2) et pEL066 (site intergénique 3) à la place du plasmide pEL079 dans les exemples 8 à 14 pour construire les plasmides donneurs spécifiques.

### Exemple 17 : Préparation d'un vaccin selon l'invention : La préparation des vaccins selon l'invention peut

se faire par toute technique usuelle connue de l'homme de l'art, par exemple par culture en flacon roulant. Des flacons roulants (175 cm²), ensemencés avec 200.10⁶ cellules primaires d'embryon de poulet, sont inoculés après 24 heures d'incubation à 37°C avec 1 ml d'une solution virale de virus HVT recombinant ayant un titre de 10⁵ pfu/ml. Après une incubation de 4 jours à 37°C, le surnageant est éliminé, les cellules sont dissociées avec une solution de trypsine versène, puis récoltées. Les cellules infectées sont alors centrifugées. Le surnageant est éliminé et les cellules sont reprises par 20 ml d'une solution contenant un stabilisateur pour lyophilisation (par exemple SPGA saccharose, phosphate, glutamate, albumine). Ce mélange est ensuite soniqué, réparti dans des flacons à raison de fractions de 1 ml et enfin lyophilisé.

Si nécessaire, le vaccin peut également être réparti et congelé au lieu d'être lyophilisé.

### Exemple 18 :

Un candidat vaccin recombinant obtenu selon l'invention et exprimant la protéine VP2 du virus de la maladie Gumboro a été utilisé pour immuniser par voie intramusculaire des poussins de 1 jour. Ces poussins ont ensuite été éprouvés à l'âge de 21 jours avec le virus de la maladie de Gumboro. Les résultats de protection ont été évalués 11 jours après épreuve en comparant les lésions de la bourse de Fabricius et la mortalité entre les groupes vaccinés et le groupe témoin non vacciné. Les résultats de ce protocole de vaccination/épreuve ont montré que le vaccin obtenu selon l'invention permet d'obtenir un bon niveau de protection.

## Revendications

1. Utilisation d'un virus HVT (Herpes Virus of Turkeys) recombinant comprenant au moins une séquence nucléotidique codant pour, et exprimant, un antigène d'un agent pathogène aviaire, insérée dans l'une des zones intergéniques 1, 2 et 3 du fragment BamHI I du génome du virus HVT, ces zones intergéniques, par référence à la souche HVT particulière qui possède la séquence nucléotidique de la figure 1, correspondant aux nucléotides de la figure 1 1213 à 1383 pour l'intergène 1, 1942 à 2283 pour l'intergène 2 et 3082 à 3569 pour l'intergène 3, pour la production d'un vaccin vivant recombinant aviaire destiné à la vaccination *in ovo,* des poussins d'un jour ou des adultes, contre ledit agent pathogène aviaire

2. Utilisation selon la revendication 1, dans laquelle l'agent pathogène aviaire est le virus de la maladie de Gumboro (IBDV).

3. Utilisation selon la revendication 1, dans laquelle l'agent pathogène aviaire est le virus de la maladie de Marek.

4. Utilisation selon la revendication 1, dans laquelle l'agent pathogène aviaire est le virus de la maladie de Newcastle (NDV).

5. Utilisation selon la revendication 1, dans laquelle l'argent pathogène aviaire est le virus de la bronchite infectieuse (IBV).

6. Utilisation selon la revendication 1, dans laquelle l'agent pathogène aviaire est le virus de la laryngotrachéite infectieuse (ILTV).

7. Utilisation selon la revendication 1, dans laquelle l'agent pathogène aviaire est le virus de l'anémie aviaire (CAV).

8. Utilisation selon la revendication 2, dans laquelle la séquence nculéotidique insérée est une séquence nucléotidique codant pour le polypeptide VP2 du virus IBDV.

9. Utilisation selon la revendication 2, dans laquelle la séquence nucléotidique insérée code pour VP3 ou pour VP2 + VP4 + VP3 du virus IBDV.

10. Utilisation selon la revendication 3, dans laquelle la séquence nucléotidique code pour gB, gC, gD ou gH + gL du virus de la maladie de Marek.

11. Utilisation selon la revendication 4, dans laquelle la séquence nucléotidique code pour F ou HN du virus de la maladie de Newcastle.

12. Utilisation selon la revendication 5, dans laquelle la séquence nucléotidique code pour S ou M du virus de la bronchite infectieuse.

13. Utilisation selon la revendication 6, dans laquelle la séquence nucléotidique code pour gB, gC, gD ou gH + gL du virus de la laryngotrachéite infectieuse.

14. Utilisation selon la revendication 7, dans laquelle la séquence nucléotidique code pour VP1 (52 kDa) + VP2 (24 kDa) du virus de l'anémie aviaire.

15. Utilisation selon l'une quelconque des revendications 1 à 14, dans laquelle ladite séquence nucléotidique est insérée sous le contrôle du promoteur CMV immediate early.

16. Utilisation selon la revendication 15, dans laquelle le promoteur CMV immediate early est le promoteur humain HCMV IE ou le promoteur murin MCMV IE.

17. Utilisation selon l'une quelconque des revendications 1 à 14, dans laquelle ladite séquence nucléotidique est insérée sous le contrôle du promoteur Marek RNA1.8.

18. Utilisation selon l'une quelconque des revendications 1 à 17, dans laquelle on utilise de 10 à 10⁴ pfu du virus HVT recombinant par dose de vaccin.

## Claims

1. Use of a recombinant HVT (herpes virus of turkeys) comprising at least one nucleotide sequence coding for and expressing an antigen of a pathogenic avian agent, inserted into one of intergen zones 1, 2 and 3 of the BamHI I fragment of the genome of the HVT virus, said intergene zones, with reference to the particular HVT strain which has the nucleotide sequence of figure 1, corresponding to nucleotides 1213 to 1383 in figure 1 for intergene 1, 1942 to 2283 for intergene 2 and 3082 to 3569 for intergene 3, for the production of a live recombinant avian vaccine intended for *in ovo* vaccination of one day old chicks and adults against said pathogenic avian agent.

2. Use according to claim 1, wherein the pathogenic avian agent is infectious bursal disease virus (IBDV).

3. Use according to claim 1, wherein the pathogenic avian agent is Marek's disease virus.

4. Use according to claim 1, wherein the pathogenic avian agent is Newcastle disease virus (NDV).

5. Use according to claim 1, wherein the pathogenic avian agent is infectious bronchitis virus (IBV).

6. Use according to claim 1, wherein the pathogenic avian agent is infectious laryngotracheitis virus (ILTV).

7. Use according to claim 1, wherein the pathogenic avian agent is avian anaemia virus (CAV).

8. Use according to claim 2, wherein the inserted nucleotide sequence is a nucleotide sequence coding for the VP2 polypeptide of IBDV.

9. Use according to claim 2, wherein the inserted nucleotide sequence codes for VP3 or for VP2 + VP4 + VP3 of IBDV.

10. Use according to claim 3, wherein the nucleotide sequence codes for gB, gC, gD or gH + gL of Marek's disease virus.

11. Use according to claim 4, wherein the nucleotide sequence codes for F or HN of the Newcastle disease virus.

12. Use according to claim 5, wherein the nucleotide sequence codes for S or M of the infectious bronchitis virus.

13. Use according to claim 6, wherein the nucleotide sequence codes for gB, gC, gD or gH + gL of the infectious laryngotracheitis virus.

14. Use according to claim 7, wherein the nucleotide sequence codes for VP1 (52 kDa) + VP2 (24 kDa) of the avian anaemia virus.

15. Use according to any one of claims 1 to 14, wherein said nucleotide sequence is inserted under the control of the CMV immediate early promoter.

16. Use according to claim 15, wherein the CMV immediate early promoter is the human HCMV IE or murine MCMV IE promoter.

17. Use according to any one of claims 1 to 14, wherein said nucleotide sequence is inserted under the control of the Marek RNA1.8 promoter.

18. Use according to any one of claims 1 to 17, wherein 10 to 10⁴ pfu of recombinant HVT virus is used per dose of vaccine.

## Patentansprüche

1. Verwendung eines rekombinanten HVT-Virus (Herpes Virus of Turkeys), das wenigstens eine Nukleotidsequenz umfasst, die für ein Antigen eines aviären pathogenen Agens kodiert und dieses exprimiert, und die in einen der intergenen Bereiche 1, 2 und 3 des BamHI I-Fragmentes des Genoms des HVT-Virus eingefügt wurde, wobei diese intergenen Bereiche, bezogen auf den besonderen HVT-Stamm mit der Nukleotidsequenz der Figur 1, den Nukleotiden 1213 bis 1383 der Figur 1 für das Intergen 1, den Nukleotiden 1942 bis 2283 für das Intergen 2 und den Nukleotiden 3082 bis 3569 für das Intergen 3 entsprechen, für die Herstellung eines aviären, rekombinanten Lebendimpfstoffs, der für die Impfung *in ovo,* der ein Tag alten Küken oder der ausgewachsenen Tiere gegen das aviäre pathogene Agens bestimmt ist.

2. Verwendung nach Anspruch 1, wobei das aviäre pathogene Agens das Virus der Gumboro-Krankheit (IBDV) ist.

3. Verwendung nach Anspruch 1, wobei das aviäre pathogene Agens das Virus der Marek-Krankheit ist.

4. Verwendung nach Anspruch 1, wobei das aviäre pathogene Agens das Virus der Newcastle-Krankheit (NDV) ist.

5. Verwendung nach Anspruch 1, wobei das aviäre pathogene Agens das Virus der Infektiösen Bronchitis (IBV) ist.

6. Verwendung nach Anspruch 1, wobei das aviäre pathogene Agens das Virus der Infektiösen Laryngotracheitis (ILTV) ist.

7. Verwendung nach Anspruch 1, wobei das aviäre pathogene Agens das Virus der Aviären Anämie (CAV) ist.

8. Verwendung nach Anspruch 2, wobei die eingefügte Nukleotidsequenz eine Nukleotidsequenz ist, die für das Polypeptid VP2 des IBDV-Virus kodiert.

9. Verwendung nach Anspruch 2, wobei die eingefügte Nukleotidsequenz für VP3 oder für VP2 + VP4 + VP3 des IBDV-Virus kodiert.

10. Verwendung nach Anspruch 3, wobei die Nukleotidsequenz für gB, gC, gD oder gH + gL des Virus der Marek-Krankheit kodiert.

11. Verwendung nach Anspruch 4, wobei die Nukleotidsequenz für F oder HN des Virus der Newcastle-Krankheit kodiert.

12. Verwendung nach Anspruch 5, wobei die Nukleotidsequenz für S oder M des Virus der Infektiösen Bronchitis kodiert.

13. Verwendung nach Anspruch 6, wobei die Nukleotidsequenz für gB, gC, gD oder gH + gL des Virus der Infektiösen Laryngotracheitis kodiert.

14. Verwendung nach Anspruch 7, wobei die Nukleotidsequenz für VP1 (52 kDa) + VP2 (24 kDa) des Virus der Aviären Anämie kodiert.

15. Verwendung nach einem der Ansprüche 1 bis 14, wobei die Nukleotidsequenz unter der Kontrolle des CMV Immediate Early-Promotors eingefügt wurde.

16. Verwendung nach Anspruch 15, wobei der CMV Immediate Early-Promotor der humane HMCV IE-Promotor oder der murine MCMV IE-Promotor ist.

17. Verwendung nach einem der Ansprüche 1 bis 14, wobei die Nukleotidsequenz unter der Kontrolle des Marek RNA1.8-Promotors eingefügt ist.

18. Verwendung nach einem der Ansprüche 1 bis 17, wobei pro Impfdosis eine PFU von 10 bis 10⁴ des rekombinanten HVT-Virus verwendet wird.


### SEQUENCE LISTING

<110> MERIAL
<120> vaccin vivant recombinant aviaire, utilisant comme vecteur un virus herpes aviaire
<130> HVT BamHI I
<140> EP 03/025194.6
   <141> 2003-11-04
<150> EP 95/402970.8
   <151> 1995-12-28
<150> FR 94/16017
   <151> 1994-12-30
<160> 28
<170> PatentIn version 3.2
<210> 1
   <211> 5838
   <212> DNA
   <213> turkey herpesvirus
<220>
   <221> misc_feature
   <222> (1030)..(1030)
   <223> n is a, c, g, or t
<220>
   <221> misc_feature
   <222> (1178)..(1178)
   <223> n is a, c, g, or t
<400> 1
   ggatccatca gcaatgcggg ctgtagtccc gattcccgtt tcaaatgaag gtgctccaac     60
   acggtcttca aagcaaccgg cataccagca aacacagact gcaactcccc gctgcaatga    120
   ttggttataa acagtaatct gtcttctgga agtatatttc gcccgacaat ccacggcgcc    180
   cccaaagtta aaaaccatcc atgtgtattt gcgtcttctc tgttaaaaga atattgactg    240
   gcattttccc gttgaccgcc agatatccaa agtacagcac gatgttgcac ggacgacttt    300
   gcagtcacca gccttccttt ccaccccccc accaacaaaa tgtttatcgt aggacccata    360
   tccgtaataa ggatgggtct ggcagcaacc ccataggcgc ctcggcgtgg tagttctcga    420
   ggatacatcc aaagaggttg agtattctct ctacacttct tgttaaatgg aaagtgcatt    480
   tgcttgttct tacaatcggc ccgagtctcg ttcacagcgc ctcgttcaca cttaaaccac    540
   aaatagtcta caggctatat gggagccaga ctgaaactca catatgacta atattcgggg    600
   gtgttagtca cgtgtagccc attgtgtgca tataacgatg ttggacgcgt ccttattcgc    660
   ggtgtacttg atactatggc agcgagcatg ggatattcat cctcgtcatc gttaacatct    720
   ctacgggttc agaatgtttg gcatgtcgtc gatcctttgc ccatcgttgc aaattacaag    780
   tccgatcgcc atgaccgcga taagcctgta ccatgtggca ttagggtgac atctcgatca    840
   tacattataa gaccaacgtg cgagtcttcc aaagacctgc acgccttctt cttcggattg    900
   tcaacgggtt cttcagaatc tatgcccata tctggcgttg agaccattgt gcgtttaatg    960
   aacaataaag cggcatgcca tggaaaggag ggctgcagat ctccattttc tcacgccact   1020
   atcctggacn ctgtagacga taattatacc atgaatatag agggggtatg tttccactgc   1080
   cactgtgatg ataagttttc tccagattgt tggatatctg cattttctgc tgccgaacaa   1140
   acttcatcgc tatgcaaaga gatgcgtgtg tacacgcngc cgttgagtat acgggaaact 1200
   aaatgttcat agaggtcttt gggctatatg ttattaaata aaataattga ccagtgaaca 1260
   atttgtttaa tgttagttta ttcaatgcat tggttgcaaa tattcattac ttctccaatg 1320
   ccaggtcatt ctttagcgag tgatgttatg acattgctgt gaaaattact acaggatata 1380
   tttttaagat gcaggagtaa caatgtgcat agtaggcgta gttatcgcag acgtgcaacg 1440
   cttcgcattt gagttaccga agtgcccaac agtgctgcgg ttatggttta tgcgcacaga 1500
   atccatgcat gtcctaattg aaccatccga tttttctttt aatcgcgatc gttgtttggg 1560
   caactgcgtt atttcagatc taaaaaattt accctttatg accatcacat ctctctggct 1620
   cataccccgc ttggataaga tatcatgtag attccgccct aagaaatgca aactaacatt 1680
   attgtcggtt ccatatacac ttccatcttg tccttcgaaa ataacaaact cgcgcaatag 1740
   accgtccgta catgcatggc cgatgtgtgt caacatcatt ggtctgctag atcccgatgg 1800
   gacgaatcgt acagtcgtcg ctccagcatt ggcaaaaatc cccagatacc ctccatgcgg 1860
   caaatctaaa ttgcgacccc gaagagactg caccaaagtc ttatcgacgc acgctgattt 1920
   ttttgaacag cgggagccca ttatcttcag tggagcgtag acgggcgagg ctaattatgt 1980
   gacatagcaa cactgcatgt atgtttttat aaatcaataa gagtacataa tttattacgt 2040
   atcatttccg tttgtaatat actgtataca tcatccacac tattagtcag cactagcgcg 2100
   cgggcgcacg ttacaatagc agcgtgcccg ttatctatat tgtccgatat ttacacataa 2160
   catttcatcg acatgattaa atacctaagt actgcacaca gatgtttaat gtatatcgtc 2220
   atataaatta tatcgctagg acagacccaa acgaccttta tcccaaacag tcagatcctc 2280
   ttctcaagtg tcgatttctg ttatggaata tgcataccct ggcccagaaa ttgcacgcac 2340
   gagcgtagtg aatgcgtcat tggttttaca tttaaaggct aaatgcacaa attctttaga 2400
   cgacagcaca tcgttaaata gcatctctag cgttcttatg aatgctaagc attggagtcc 2460
   tcctggtcgg ccacaataac agctgagtat cataccctga gctccggggt tgtcgcacat 2520
   agcggattcg tataaacata ggattttccg cgaatccatc agttgcaaaa atctgttagg 2580
   ctccatcaac aacgctggat ttacttcaga tccacgcgta aagtaatggt gctcgaatac 2640
   cgtttttaga gttgtcggca tttcaaggaa caaagaattc atttcttcat tgcaacgacg 2700
   cgccagaaat cccaagacct ctttgggtag tatgttcttg cctataaaac acggcgttcc 2760
   aagtgccagg aaccacgcat gtgttactgt tggggcgtat tcagaaataa agcggggttt 2820
   atgcggcttt tgaagctcgg atatccaaag tatcgcttgc tgatgaacga gcgatgtagc 2880
   tgttacaaaa cctcctttcc atcctccagt caacataata tttatcggcc tacctatgtc 2940
   cgtaataagt attggtcggg caattattcc gtatgaggtc ttgcaggaat aagctcttag 3000
   ggacagccag cttggatatg gtgcgaaaca gaccttctcg gcttcagaat gtcgctccgc 3060
   agtctcttcg tgtcggtgca tcttagatcc accatcaatg tgtgcagcat tgactcccgc 3120
   ccgtcgaata ttccttttgt tacgatgcag taatgagcac gatcatgggc ggggcgatga 3180
   cgttctattt gcatgtctgc gaacaatttg cgtcagtcat acagctatgg agtgggccat 3240
   ttctggcgtc aacttaaaaa cgcgaaccgc agacatatgt atttgcatgc aaagacgtat 3300
   cttcgtattt ctgggcatct tcaaatgctc tggccaatat ggcaatgaat ttggattcgt 3360
   ttgacgccga tggtatgcag tgcaaatgtg ccaatagccc acatccgaaa aagttatttg 3420
   tcatacaagc aggtgttaag tagcaatcac ataaaggcac cagacgcctc atggcatcat 3480
   aatgaatagc tccttctccc cactggaacc actgacaaaa tctgcgagta tattccgcaa 3540
   accacatttt atttctcata gaaactaccc taaatccttt taacggggaa gaagaatcct 3600
   agatagtgct tgaagtcatg actgttactg ctgcaataac actgtatatt atttataaat 3660
   tccgtttgtc taggtatctg atgtaggcat tccgatccct ttactattgc gtcttcacga 3720
   ccaaatggga atgcgccaaa atccccacac ctcatcaccc tggaggcaga ttgtgtatta 3780
   ttaatatccg ccgattgaag cacaaaacgg tacggtactg ttcctaattc tggtatagat 3840
   tctatggtca aaagtctgca tatccccgac attgccatga gatcacacag tccaagtagc 3900
   atgtttattg agtcactcag actgtcaacg tccctcgccg caccaccaat cgaaaataaa 3960
   gtatctacgc aagttatagc tccgcatttt ctatcgctag cagcaatcgc gacgcaaaac 4020
   ataaaggcca tgttgggatt tgaactctct ggggggcttg ttatcttctg caccgtcgca 4080
   gtcgcagttt tccgaaattt atgtctaata tattttccgg ccgtgctcca atcggccgaa 4140
   aagaatctgc gtattaccag actcattgac gggccgataa agaccataaa acaaaattcc 4200
   tgtgcactcc ctcctccagt tttgccatcg tccaagtccc gtaacttttt ttgcgtttcg 4260
   aggagcaagc gttcgttatc cctacccaca cttgttttcc accgttttct tattataagc 4320
   ggttgtatcg ccaacgcgtc accgcaggtt gtcacataca gtgatggcat acttgaacgt 4380
   gcaacaacgc gctcgctttg caaatctaag tcattgacca tcaaatcgcg ttgagaggat 4440
   agccaggcat cttttttcct agtatggtga cggtgcagcc accccaactc agttcttgta 4500
   aaaaaagcta ttggcgggaa tttatgttct gaggtgcatt ctatatttat gagtccatca 4560
   aatgccatta accagattcg tattttttcg ctcgacccgg catcactatg gatacaatac 4620
   ctttctatgg cccatttcag ctctcgaacc aaccacacgg acaattgact aacataagta 4680
   tgatctttat cacagtcgca cccatctgag ttatatttat ggcatccgag cgctcttact 4740
   gtacggtcgg atacacccat ggtttttcct ttatatagtc gggttatagt ctgtcgggtt 4800
   tggcggtagc acggagtagt ttgattttta agaatcgaaa accggcttgg agagaccact 4860
   gtcgaatatt tgtccgtata ctctacacgt gagtgttgtc cattcctagg tatattcatc 4920
   tgttcggata ccttcaattg ctgttcaggc ataaccttaa agcatatgtt atgttgtaca 4980
   tcaaaacttg gtgagttatg ttcgattgcc gcgcataaag aatcgtacat gagcgtttct 5040
   gctaacatac tatctatatt ctcacacgcc cctgcatata ctgttcctat tccaaattca 5100
   cgttttgccc catcggctat ctgctcccaa aaagttgtaa tataggtgcc gctgggtgcg 5160
   aaattttcat cagttgtatt cctgataaac tgaatcactt tacataattt ttgccacata 5220
   tctgcgtgca gccatagtat cgaacccgtg ggctcggaga cgacagtgcg tacaatgggt 5280
   attttacctt tccccaacaa aataatggta tacaagttag gtccgtacct agaccttaat 5340
   gtttccaatt cttctgaatc actgcactct cgtaggggag taacggtaat aatttcgtct 5400
   ctgagccccg ttttgcgttg aaaactaatc acattagata atgtgcaatc ggtttctttt 5460
   atccggatac atctaagtat tatgacatcg gtggtcattg tttccatcaa cgaccatctt 5520
   ttacgatcgc ccatactact catggacgtt gtcggtgttg aaaaatcacc agaattgcaa 5580
   cggatctctg ggtaccatgc tgctgatgga attggcggtt ttaattgttg tttcagtcta 5640
   ttattgctat ctttggcggg gttgaataat gtggggggag agtgattgca ggaatccgaa 5700
   tgggtcaata aaacgaccgt gctccgttct gccggcgccg atccgattga agctatatac 5760
   ttcgcttctc tccccacttt tccaatttga tccggaaata aaacggcccc ggacaacagt 5820
   atcgtacgat ccggatcc 5838
<210> 2
   <211> 24
   <212> DNA
   <213> Artificial
<220>
   <223> oligonucléotide servant d'amorce de PCR
<400> 2
   cattataaga ccaacgtgcg agtc 24
<210> 3
   <211> 31
   <212> DNA
   <213> Artificial
<220>
   <223> oligonucléotide servant d'amorce de PCR
<400> 3
   gttcacgtcg acaattattt tatttaataa c 31
<210> 4
   <211> 23
   <212> DNA
   <213> Artificial
<220>
   <223> oligonucléotide servant d'amorce de PCR
<400> 4
   aagataatgg gctcccgctg ttc 23
<210> 5
   <211> 42
   <212> DNA
   <213> Artificial
<220>
   <223> oligonucléotide servant d'amorce de PCR
<400> 5
   taattgtcga ccccggggaa ttcgtttaat gttagtttat tc 42
<210> 6
   <211> 24
   <212> DNA
   <213> Artificial
<220>
   <223> oligonucléotide servant d'amorce de PCR
<400> 6
   gaaatgcaaa ctaacattat tgtc 24
<210> 7
   <211> 31
   <212> DNA
   <213> Artificial
<220>
   <223> oligonucléotide servant d'amorce de PCR
<400> 7
   gtgtaaatag tcgacaatat agataacggg c 31
<210> 8
   <211> 43
   <212> DNA
   <213> Artificial
<220>
   <223> oligonucléotide servant d'amorce de PCR
<400> 8
   ctatattgtc gaccccgggg aattcatcga catgattaaa tac 43
<210> 9
   <211> 35
   <212> DNA
   <213> Artificial
<220>
   <223> oligonucléotide servant d'amorce de PCR
<400> 9
   caatgaagaa atattttctt tgttccttga aatgc 35
<210> 10
   <211> 23
   <212> DNA
   <213> Artificial
<220>
   <223> oligonucléotide servant d'amorce de PCR
<400> 10
   ccgaattcat ataagcttac gtg 23
<210> 11
   <211> 31
   <212> DNA
   <213> Artificial
<220>
   <223> oligonucléotide servant d'amorce de PCR
<400> 11
   tcgacacgta agcttatatg aattcggcat g 31
<210> 12
   <211> 50
   <212> DNA
   <213> Artificial
<220>
   <223> oligonucléotide servant d'amorce de PCR
<400> 12
   cgaattcact agtgtgtgtc tgcaggcggc cgcgtgtgtg tcgacggtac 50
<210> 13
   <211> 50
   <212> DNA
   <213> Artificial
<220>
   <223> oligonucléotide servant d'amorce de PCR
<400> 13
   cgtcgacaca cacgcggccg cctgcagaca cacactagtg aattcgagct 50
<210> 14
   <211> 40
   <212> DNA
   <213> Artificial
<220>
   <223> oligonucléotide servant d'amorce de PCR
<400> 14
   gactggtacc gcggccgcat gcacttttta ggcggaattg 40
<210> 15
   <211> 18
   <212> DNA
   <213> Artificial
<220>
   <223> oligonucléotide servant d'amorce de PCR
<400> 15
   ttcgggacat tttcgcgg 18
<210> 16
   <211> 18
   <212> DNA
   <213> Artificial
<220>
   <223> oligonucléotide servant d'amorce de PCR
<400> 16
   tatatggcgt tagtctcc 18
<210> 17
   <211> 41
   <212> DNA
   <213> Artificial
<220>
   <223> oligonucléotide servant d'amorce de PCR
<400> 17
   ttgcgagctc gcggccgctt attacacagc atcatcttct g 41
<210> 18
   <211> 2521
   <212> DNA
   <213> Newcastle disease virus
<400> 18
   tgctacctga tgtacaagca aaaggcacaa caaaagacct tgttatggct tgggaataat 60
   acccttgatc agatgagagc cactacaaaa atatgaatac aaacgagagg cggaggtatc 120
   cccaatagca atttgcgtgt aaattctggc aacctgttaa ttagaagaat taagaaaaaa 180
   ccactggatg taagtgacaa acaagcaata cacgggtaga acggtcggag aagccacccc 240
   tcaatcggga atcaggcctc acaacgtcct ttctaccgca tcatcaatag cagacttcgg 300
   tcatggaccg tgcagttagc agagttgcgc tagagaatga agaaagagaa gcaaagaata 360
   catggcgctt tgtattccgg attgcaatct tacttttaat agtaacaacc ttagccatct 420
   ctgcaaccgc cctggtatat agcatggagg ctagcacgcc tggcgacctt gttggcatac 480
   cgactatgat ctctaaggca gaagaaaaga ttacatctgc actcagttct aatcaagatg 540
   tagtagatag gatatataag caggtggccc ttgagtctcc attggcgttg ctaaacactg 600
   aatctgtaat tatgaatgca ataacgtctc tctcttatca aatcaatgga gctgcaaata 660
   atagcgggtg tggggcacct gttcatgacc cagattatat cggggggata ggcaaagaac 720
   ttattgtgga tgacgctagt gatgtcacat cattctatcc ctctgcgttc caagaacacc 780
   tgaactttat cccggcacct actacaggat caggttgcac tcggataccc tcattcgaca 840
   taagcgctac ccactactgt tacactcaca atgtgatatt atctggttgc agagatcact 900
   cacactcata tcagtactta gcacttggcg tgcttcggac atctgcaaca gggagggtat 960
   tcttttctac tctgcgttcc atcaatttgg atgacagcca aaatcggaag tcttgcagtg 1020
   tgagtgcaac tcccttaggt tgtgatatgc tgtgctctaa aatcacagag actgaggaag 1080
   aggattatag ttcaattacg cctacatcga tggtgcacgg aaggttaggg tttgacggtc 1140
   aataccatga gaaggactta gacgtcataa ctttatttaa ggattgggtg gcaaattacc 1200
   caggagtggg gggtgggtct tttattaaca accgcgtatg gttcccagtc tacggagggc 1260
   taaaacccaa ttcgcctagt gacaccgcac aagaagggag atatgtaata tacaagcgct 1320
   acaatgacac atgcccagat gaacaagatt accagattcg gatggctaag tcttcatata 1380
   agcctgggcg gtttggtgga aaacgcgtac agcaggccat cttatctatc aaggtgtcaa 1440
   catctttggg cgaggacccg gtgctgactg taccgcctaa tacaatcaca ctcatggggg 1500
   ccgaaggcag agttctcaca gtagggacat ctcatttctt gtaccagcga gggtcttcat 1560
   acttctctcc tgctttatta taccctatga cagtcaacaa caaaacggct actcttcata 1620
   gtccttacac attcaatgct ttcactaggc caggtagtgt cccttgtcag gcatcagcaa 1680
   gatgccccaa ctcatgtgtc actggagttt atactgatcc gtatccctta gtcttccata 1740
   ggaaccatac cttgcggggg gtattcggga caatgcttga tgatgaacaa gcaagactta 1800
   accctgtatc tgcagtattt gataacatat cccgcagtcg cataacccgg gtaagttcaa 1860
   gccgtactaa ggcagcatac acgacatcga catgttttaa agttgtcaag accaataaaa 1920
   catattgcct cagcattgca gaaatatcca ataccctctt cggggaattc aggatcgttc 1980
   ctttactagt tgagattctc aaggatgatg ggatttaaga agcttggtct ggccagttga 2040
   gtcaactgcg agagggtcgg aaagatgaca ttgtgtcacc ttttttttgt aatgccaagg 2100
   atcaaactgg ataccggcgc gagcccgaat cctatgctgc cagtcagcca taatcagata 2160
   gtactaatat gattagtctt aatcttgtcg atagtaactt ggttaagaaa aaatatgagt 2220
   ggtagtgaga tacacagcta aacaactcac gagagatagc acgggtagga catggcgagc 2280
   tccggtcccg aaagggcaga gcatcagatt atcctaccag agtcacatct gtcctcacca 2340
   ttggtcaagc acaaactgct ctattactgg aaattaactg gcgtaccgct tcctgacgaa 2400
   tgtgacttcg accacctcat tatcagccga caatggaaga aaatacttga atcggccact 2460
   cctgacactg agaggatgat aaagctcggg cgggcagtac accagactct cgaccaccgc 2520
   c 2521
<210> 19
   <211> 22
   <212> DNA
   <213> Artificial
<220>
   <223> oligonucléotide servant d'amorce de PCR
<400> 19 22
   cagaccaagc ttcttaaatc cc 22
<210> 20
   <211> 16
   <212> DNA
   <213> Artificial
<220>
   <223> oligonucléotide servant d'amorce de PCR
<400> 20
   gtattcggga caatgc 16
<210> 21
   <211> 21
   <212> DNA
   <213> Artificial
<220>
   <223> oligonucléotide servant d'amorce de PCR
<400> 21
   gtgacatcac tagcgtcatc c 21
<210> 22
   <211> 36
   <212> DNA
   <213> Artificial
<220>
   <223> oligonucléotide servant d'amorce de PCR
<400> 22
   ccgcatcatc agcggccgcg atcggtcatg gacagt 36
<210> 23
   <211> 18
   <212> DNA
   <213> Artificial
<220>
   <223> oligonucléotide servant d'amorce de PCR
<400> 23
   tgaccctgtc tgggatga 18
<210> 24
   <211> 36
   <212> DNA
   <213> Artificial
<220>
   <223> oligonucléotide servant d'amorce de PCR
<400> 24
   ggatcccggt cgacacattg cggccgcaag atgggc 36
<210> 25
   <211> 800
   <212> DNA
   <213> Marek's disease gammaherpesvirus MKT-1
<400> 25
   gaattccatc accccctgcc gatcttgcac gcggggacga gcaaagcgtg cggtgcgggc 60
   agaaagacaa ggatggctgt gggttgaaag atgaaaaaca aatcgcggtt gtgggtcatg 120
   agtggaggga gggtgccatc tgtgatgccg agaggtcaaa ctatgttata aagaaaaacg 180
   atgggtggga aatataataa agcaaccgaa atggtacata aaaactaaaa atacctacac 240
   ggttacacca ccgatcaggc gaagaagttc caaacgatta acaaccggga cgagacgttg 300
   ccgttcgatc caggtctctg cttttttgta tctcttatcc tataccgccg cctcccgtcc 360
   gacgagagca agtcgcaccg ccactcgagg ccacaagaaa ttacgattct tatacgggtg 420
   ggcgtaccgc ctactcgaac tatcacgtga tgtgtatgca aatgagcagt gcgaacgcgt 480
   cagcgttcgc actgcgaacc aataatatat tatattatat tatattattg gactctggtg 540
   cgaacgccga ggtgagccaa tcggatatgg cgatatgtta tcacgtgaca tgtaccgccc 600
   caaattcgca cttgagtgtt gggggtacat gtgggggcgg ctcggctctt gtgtataaaa 660
   gagcggcggt tgcgaggttc cttctctctt cgcgatgctc tctcagaatg gcacggccga 720
   tcccccatat atttcctgaa ggaacgcata gctaggcgac gaacgagctg aatttctccc 780
   ttcatcaaat aagtaataaa 800
<210> 26
   <211> 32
   <212> DNA
   <213> Artificial
<220>
   <223> oligonucléotide servant d'amorce de PCR
<400> 26
   ggtctactag tattggactc tggtgcgaac gc 32
<210> 27
   <211> 31
   <212> DNA
   <213> Artificial
<220>
   <223> oligonucléotide servant d'amorce de PCR
<400> 27
   gtccagaatt cgcgaagaga gaaggaacct c 31
<210> 28
   <211> 33
   <212> DNA
   <213> Artificial
<220>
   <223> oligonucléotide servant d'amorce de PCR
<400> 28
   gtgtcctgca gtcgcgaaga gagaaggaac ctc 33